# EUROPEAN PATENT SPECIFICATION

(11) **EP 3 170 416 B1**
(45) Date of publication and mention of the grant of the patent: **25.11.2020**
(21) Application number: 16166565.8
(22) Date of filing: 22.04.2016
(51) Int. Cl.: A43B 1/00, A43B 17/10, A61K 36/9064, A61K 36/232, A61K 36/236, A61K 36/61, A43B 7/00, A61M 35/00, A61P 1/10, A61P 3/10, A61P 7/06, A61P 9/12, A61P 11/06, A61P 13/12, A61P 17/04, A61P 19/08, A61P 25/20, A61P 29/00

(54) **THE BODY SELF-HEALING AND RECUPERATING SHOES HAVING A MEDICINAL MAGNETIC THERAPEUTIC FUNCTION**
SCHUHE ZUR KÖRPERSELBSTHEILUNG UND -WIEDERHERSTELLUNG MIT EINER MEDIZINISCHEN MAGNETTHERAPEUTISCHEN FUNKTION
CHAUSSURES DE RÉCUPÉRATION ET AUTOGUÉRISON DU CORPS AYANT UNE FONCTION THÉRAPEUTIQUE MAGNÉTIQUE MÉDICINALE

(30) Priority: 20.11.2015 CN 201510819836
(43) Date of publication of application: 24.05.2017
(73) Proprietor: Wu, Liji, Inner Mongolia 010011 (CN)
(72) Inventor: WU, Liji, 010011 Hohhot City Inner Mongolia (CN)
(74) Representative: Michalski Hüttermann & Partner Patentanwälte mbB

(56) References cited:
- EP-A1- 2 277 400
- EP-A1- 2 311 340
- EP-A1- 3 135 145
- US-A- 6 151 807
- DATABASE WPI Week 200437 Thomson Scientific, London, GB; AN 2004-390836 XP002767593, -& CN 1 476 792 A (WU L) 25 February 2004 (2004-02-25)
- DATABASE WPI Week 200055 Thomson Scientific, London, GB; AN 2000-579715 XP002767594, -& CN 1 142 341 A (WU L) 12 February 1997 (1997-02-12)
- DATABASE WPI Week 200327 Thomson Scientific, London, GB; AN 2003-269009 XP002767595, -& CN 1 171 222 A (WU L) 28 January 1998 (1998-01-28)
- DATABASE WPI Week 200319 Thomson Scientific, London, GB; AN 2003-184737 XP002767596, -& CN 1 163 083 A (WU L) 29 October 1997 (1997-10-29)
- ZHENG Y: "Odor destroying insole drug formula used for preventing and eliminating dermatophytosis, comprises Rheum officinale, saponin, magnolia flower, Ligusticum wallichii, plantain, Agastache rugosus, Mentha haplocalyx, and golden cypress", WPI / THOMSON,, vol. 2012, no. 51, 9 May 2012 (2012-05-09) , XP002765794,

## Description

### TECHNICAL FIELD

The present invention relates to a pair of body self-healing and recuperating shoes having a medicinal magnetic therapeutic function, including the shoes suitable for wearing during spring or fall seasons, sandals, slippers, cotton-padded shoes, and other various types of shoes.

### BACKGROUND

CN 1476792A disclosed a pair of magnetic therapy shoes with mongolian medicines inside. The medicine in the medicine box are the mixture of the volatile oil extracted from clove, woody, sandalwood, asparagus, Amomum villosum, bean curd, dried bean curd, grass bean curd, grass fruit, turmeric, curcuma, puzzle and benzoin, borneol, camphor, a total of 19 Chinese and Mongolian drug. The magnetic therapy pieces are arranged at local positions on a shoe corresponding to a part of the acupuncture point of the foot. And the magnetic therapy pieces are the wafer with a diameter of 3-20mm and a thickness of 1-5mm.

CN1142341A disclosed a pair of medicine magnetic health care shoes. The medicine in shoes is a medicine powder or a medicine liquid which can improve the blood circulation of the human body and keep body healthy, the said medicine is comprised of Millettia, Wu Chia Pee, barley, Artemisia leaf, taro, stone calamus, mint and borneol Starch, clove, angelica, angelica, ginseng, marshes, mint, red peony, nong xiang and as arum. The permanent magnet pieces are arranged at local positions on a shoe corresponding to a part of the acupuncture point of the foot. And the permanent magnet pieces are the wafer with a diameter of 8-13mm and a thickness of 1-2mm.

CN1171222A disclosed a pair of medicine magnetic shoes, containing Chinese medicine and Mongolian medicine preparation, which includes Evodia, incense, borneol, menthol, clove, leaves, angelica, puzzle, Amomum villosum, Su Hexiang, benzoin, sandalwood, Gan Song, patchouli, Asarum and mint. The permanent magnet pieces are arranged at local positions on a shoe corresponding to a part of the acupuncture point of the foot. And the permanent magnet pieces are the wafer with a diameter of 9-15mm and a thickness of 1-2mm or with a diameter of 5.5-9.5mm and a thickness of 1-2mm .

CN1163083A disclosed a pair of medicinal medical health care shoes, containing traditional Chinese medicine and Mongolian medicine preparation, which including Evodia, incense, borneol, Jing Jie, Salvia, clove, pepper, Artemisia leaf, horse pig, cinnamon, angelica, Mushrooms, acacia, cabbage, gardenia, patchouli, toosendan, Xinyi, hematoxylin, safflower, ferulic, Guanmutong, Fir, asarum, maple fat, mint. The permanent magnet pieces are arranged at local positions on a shoe corresponding to a part of the acupuncture point of the foot. And the permanent magnet pieces are the wafer with a diameter of 9-15mm and a thickness of 1-2mm or with a diameter of 9.5mm and a thickness of 1-2mm .

US6151807A disclosed a health care shoe, comprising: a sole having a plurality of magnet cavities specifically indented and distributed on an inner surface thereof and at least a medicine receiving cavity having at least a medicine unit received therein, wherein said medicine unit comprises a medicine layer therein and a bottom magnet element positioned thereunder so as to produce magnetic field around said medicine unit.

EP 2311340A1 disclosed a medical footwear, which comprises at least one of the following categories: powdered preparations, liquid preparations and essential oil compositions, wherein all of the said powdered preparations and liquid preparations include one component borneol, compounded with medicines including clove, amomum cardamomum, angelica, radices zedoariae, radix angelicae and lignum cinnamomi camphorae, or are prepared with any one of the above medicine compounded solely with borneol; the weight ratios of the said medicinal materials clove, amomum cardamomum, angelica, radices zedoariae, radix angelicae and lignum cinnamomi camphorae are in the range from 1 to 9, respectively.

EP 2277400A1 disclosed a kind of shoes for magnetotherapy.

ZHENG Y et. al ("Odor destroying insole drug formula used for preventing and eliminating dermatophytosis, comprises Rheum officinale, saponin, magnolia flower, Ligusticum wallichii , plantain, Agastache rugosus, Mentha haplocalyx, and golden cypress", WPI / THOMSON vol. 2012, no. 51, 9 May 2012 (2012-05-09), XP002765794) disclosed a odor destroying insole drug formula used for preventing and eliminating dermatophytosis.

However, the prior arts said above are failed to disclose the pharmaceutical composition said in the present invention and the said magnets are magnetic beads simultaneously.

The existing functional shoes are yet incapable of alleviating various symptoms of hypoxic lesions suffered by the elderly, let alone restoring the pathological indexes to normal. Furthermore, neither functional shoes have been tested by relevant clinical laboratory, nor relevant clinical inspection reports can be obtained as yet.

### SUMMARY OF THE INVENTION

The present invention is defined by the independent claims. Advantageous embodiments are given in the dependent claims.

The present invention relates to a pair of body self-healing and recuperating shoes having a medicinal magnetic therapeutic function, wherein one or more breathable containers are set up in said recuperating shoes, said containers contain: a volatile oily medicament, a non-volatile oily liquid medicament or a powdered medicament;
said volatile oily medicament comprises 1-9 parts by weight of the following raw medicines: angelica anomala oil or its extract, or clove oil or its extract, and 1-9 parts by weight of borneol corresponding to each of the raw medicine;
said powdered medicament comprises 1-9 parts by weight of the following raw medicines: ligusticum wallichii, abutilon pepper, amomum cardamom, angelica anomala and clove, and 1-9 parts by weight of borneol corresponding to each of the raw medicine;
said non-volatile oily liquid medicament comprises 1-9 parts by weight of the following raw medicines: ligusticum wallichii, abutilon pepper, amomum cardamom, angelica anomala and clove, and 1-9 parts by weight of borneol corresponding to each of the raw medicine;
one or more magnets are installed in said body self-healing and recuperating shoes; wherein said magnets are installed in the sole and/or upper and/or back counter of the shoes, and said breathable containers are located within the insole;
wherein said magnets are magnetic beads, one or more recessed grooves are cut into said shoe soles or insoles, a magnetic bead is set up in each recessed groove; the length, width and height of the recessed groove is greater than the diameter of the magnetic bead, allowing the bead to roll freely

The present invention further relates to a method of preparation of the powdered medicament like defined above, wherein:
(1) ligusticum wallichii, abutilon pepper, amomum cardamom, angelica anomala and clove, and 1-9 parts by weight of borneol corresponding to each of the raw medicine are weigh out according to the weight ratio;
(2) Pick and grind each of the above-mentioned raw materials into powders and sieve the powders to obtain the respective raw medicinal powders;
(3) Add borneol into each raw medicinal powder to obtain said single raw medicinal
powder, then the mixture of the raw medicinal powders can be obtained by mixing respective single raw medicinal powders with each other.

The present invention further relates to a method of preparation of the non-volatile oily liquid medicament like defined above, wherein:
(1) weigh out the raw materials according to the weight ratio of 1-9 parts by weight of one of the following raw medicines: angelica anomala oil or its extract, or clove oil or its extract, and 1-9 parts by weight of borneol corresponding to each of the raw medicine;
(2) Pick carefully and grind each of the above-mentioned raw materials into the powders and sieve the powders to obtain the respective raw medicinal powders;
(3) Add borneol into each raw medicinal powder to obtain said single raw medicinal powders;
(4) Soak each of said single raw medicinal powder in an acidic solution respectively, then extract the powders by diacolation to obtain extracted stock solutions of single raw medicinal powders, then mixed the single extracted stock solutions with each other

To complementing the deficiencies of the existing techniques, the present invention aims at providing a pair of advanced body self-healing and recuperating shoes to treat a number of diseases simultaneously with high cure rates, quickly, in a shorter course, simply, conveniently, safely, reliably and comfortably; after the patient wore the shoes, the Joule heating effect on the feet will cause feet warm and comfortable and cause waists and legs stronger without pain. These body self-healing and recuperating shoes having a medicinal magnetic therapeutic function are particularly suitable for those who are afraid of taking medications, injections, acupuncture, surgery, and pain, and the frail elderly, the shoes can play the role of treatment on the patients at all times and places during they walks wearing the shoes, which can greatly reduce the financial burden on patients. The shoes are characterized in the dual-use which can be worn and as a means of treatment. The technique of this invention has passed two clinical tests and the statistical reports on the results of the clinical tests have been obtained.

To achieve the above object, the present invention provides a kind of body self-healing and recuperating shoes having a medicinal magnetic therapeutic function, said the breathable containers are set up in shoes, said containers contain: a volatile oily medicament, a non-volatile oily liquid medicament or a powdered medicament, or the mixture of more than two of the above-mentioned three kinds of medicaments;

Said volatile oily medicament comprises 1-9 parts by weight of one of the following raw medicines, or the mixture of more than two of the following raw medicines: angelica anomala oil or its extract, or clove oil or its extract; and 1-9 parts by weight of borneol corresponding to each of the raw medicine;

Said powdered medicament comprises 1-9 parts by weight of one of the following raw medicines, or the mixture of more than more than two of the following raw medicines: ligusticum wallichii, abutilon pepper, amomum cardamom, angelica anomala and clove; and 1-9 parts by weight of borneol corresponding to each of the raw medicine;

Said non-volatile oily medicament comprises 1-9 parts by weight of one of the following raw medicines, or the mixture of more than two of the following raw medicines: ligusticum wallichii, abutilon pepper, amomum cardamom, angelica anomala and clove; and 1-9 parts by weight of borneol corresponding to each of the raw medicine;

The magnets are installed in said body self-healing and recuperating shoes, preferably installed in the soles and/or uppers and/or back counter of the shoes, said breathable container is preferably located within the insole.

Wherein the container is punched with holes or covered with cloth, allowing medicament components to evaporate out from holes or cloth.

Wherein said body self-healing and recuperating shoes include the shoes suitable for wearing during spring or fall seasons, sandals, slippers and cotton padded shoes.

Wherein said single volatile oily medicament is prepared by adding borneol into said raw medicines; or, when the mixture ofraw medicines is needed, it can be obtained by mixing single volatile oily medicaments with each other.

Wherein the powdered medicament can be prepared by the following method:
(1) Weigh out raw materials: weigh out the above-mentioned raw materialsaccording to the weight ratio;
(2) Pick carefully each of the above-mentioned raw materials, grind them into the powders and sieve the powders to obtain the respective raw medicinal powders;
(3) Add borneol into each raw medicinal powder to obtain said single raw medicinal powder, or, when the mixture of the raw medicinal powders is needed, it can be obtained by mixing respective single raw medicinal powders with each other.

Wherein the non-volatile oily liquid medication can be prepared by the following method:
(1) Weigh out raw materials: weigh out the above-mentioned raw materials according to the weight ratio;
(2) Pick carefully each of the above-mentioned raw materials, grind them into the powders and sieve the powders to obtain the respective raw medicinal powders;
(3) Add borneol into each raw medicinal powder to obtain said single raw medicinal powders;
(4) Soak each of said single raw medicinal powder in an acidic solution respectively, then extract individually the powders by diacolation to obtain extracted stock solutions of single raw medicinal powders; if the mixture of stock solutions is needed, it can be obtained by mixing stock solutions of single raw medicinal powders with each other; said acidic solution is preferably acetic acid.

Wherein said magnets are magnetic slices or magnetic beads, one or more recessed grooves are cut into said shoe soles or insoles, a magnetic bead is set up in each recessed groove; the length, width and height of the recessed groove is greater than the diameter of the magnetic bead, allowing the bead to roll freely.

Wherein the recessed groove can be ring-, oval ring-, rectangular-ambulatory-plane -, cross-, long strip- or triangle shaped; said oval ring-, rectangular-ambulatory-plane-, cross shaped-, long strip- or triangle-shaped recessed grooves can be arranged in longitudinal, transverse or diagonal directions.

Wherein more than one shallow recessed groove, or more than one pair of parallel shallow recessed grooves are set up in said portions of the soles or insoles in contact with the soles of the feet, as well as in the upper- and back counter of the shoes; more than one magnet or more than one pair of parallel magnets are set up in each recessed groove.

Wherein the magnets are selected from rare earth permanent magnetic materials, or various types of hard magnets containing magnetic materials and soft magnets made of rubber or plastic or other soft materials.

Said body self-healing and recuperating shoes provided by the present invention can repair and enhance human body self-healing capability and result in the self-healing of hypoxic diseases of the elderly. By means of wearing the body self-healing and recuperating shoes having a medicinal magnetic therapeutic function during the patients walks, the effects of medicaments and magnets by the transdermal absorption of the feet will increase the velocity of red cell spiral migration, dredge aged and blocked capillaries in whole of the body, promote blood circulation, and increase the oxygen carrying capacity of red cells, consequently guarantee the supply of oxygen for the whole body, as a result, hypoxidosis, the source of many diseases, is eliminated, and the body's self-healing ability is comprehensively repaired and upgraded, accordingly various hypoxic lesions and functional disorders are self-healed and recovered, such as the hypoxic hyperosteogeny of articular chondrocytes in cervical vertebrae, lumbar vertebrae, shoulders, hands, legs, feet and knees; lumbar intervertebral disc rupture, the nucleus pulposus prominent, stiff and swelling joints, limited activity, and difficulty in walking and going up and down the stairs, femoral head necrosis, disorder in the excretion of synovial fluid by joint synovial, as well as various kinds of inflammation and sore pain, cervical spondylosis, lumbar-, shoulder-, rheumatic- and rheumatoid-arthritis, diabetes, diabetic cardiovascular diseases, diabetic hypoxic bone marrow membrane impatency and anoxic hematopoietic function disorder of bone marrow stem cells, diabetic nephropathy syndrome, secondary nephrotic syndrome, severe skin itching, neurodermatitis, coronary heart disease, bradycardia, hypotension, anemia, hypertension, myocardial infarction, cerebral infarction, stroke, asthma, insomnia, constipation, benign prostatic hyperplasia etc. Even for the patients suffering from more than 10 kinds of diseases, eight kinds of diseases can be self-healed (in 3 months); two kinds of diseases can be improved very markedly (in six months).

### DESCRIPTION OF THE DRAWINGS

Figure 1 is a perspective view of the recuperating shoe;
Figure 2 is a perspective view of the shoe upper;
Figure 3 is a cross-sectional view of the insole;
Figure 4 is a perspective view of the sole;
Figure 5 is a structural view of a container;
Figure 6 is an exploded view of Figure 5.

### DETAILED DESCRIPTION OF THE EMBODIMENTS

Following are more detailed descriptions, in combination with the examples, of the above-mentioned and other technical characteristics and advantages of the present invention.

Figure 1 is a perspective view of a body self-healing and recuperating shoe, said self-healing and recuperating shoe comprises insole 11, at least one recessed groove 12 is set up in the insole 11, the container 13 or magnetic beads 15 is installed in the recessed groove 12, shoe upper 14 or back counter is provided with magnetic beads 15 or magnetic slices. Rare earth permanent magnet materials or various types of hard magnets containing magnetic materials, and soft magnets made of rubber or plastic or other soft materials can be selected as material of magnetic beads 15 or slices.

As shown in Figure 5, said container 13 is formed by snap joint of an upper lid 131 with holes and inner buckle edge and the bottom box 132 with outer buckle edge.

As shown in Figures 8 and 9, said container 13 is composed of the cramp ring 133 with inner buckle edge and the bottom box 132 with outer buckle edge, at least one layer of cloth 134 is fastened on the opening of bottom box 132 through said cramp ring 133.

Container 13 is punched with holes or is covered with at least one layer of cloth 134, allowing medication components to evaporate out from said holes or cloth 134.

The above content is only provided as a preferred embodiment, in fact, a number of adjustments may be made, for example:
The recessed groove 12 in the insole 11, and the container 13, can be designed to be round, square, heteromorphic, or any shape, volume, size using any kind of material, because the structural change is simple and easy to understand, it will not be shown graphically.

The container 13 contains the volatile oily medicament, the non-volatile oily liquid medicament or the powdered medicament, or the mixture of more than two of the three above medicaments;
The medicaments prepared in following examples are all placed in the containers in insole.

The phase II clinical reports provided by Inner Mongolia International Mongolian Hospital and Affiliated Hospital of Inner Mongolia Medical University:
Total effective rate to diabetes and lumbar spondylosis: 92%;
The total effective rate to coronary heart disease: 94%; the total effective rate to hypertension : 98%;
The total effective rate to sequelae of stroke and of cerebral infarction: 90%; to cervical spondylosis and scapulohumeral periarthritis: 96%.

For the above-mentioned phase II clinical study, the statistical reports of clinical laboratory have all been obtained by national statistical department.

### (1) Diabetes:

① Conclusions to the studies by Inner Mongolia International Mongolian Hospital and Affiliated Hospital of Inner Mongolia Medical University:
   In the two hospitals 50 diabetic patients were observed for 90 days individually: The total effective rate was 92%, cure rate 1%, obvious effective rate 17%, effective rate 74%, ineffective rate 8%, cure-remarkable-effectiveness rate(clinical control +obvious effect) 18%.
② Typical clinic cases:

### Example 1

3 parts by weight of borneol was added to 2 parts by weight of angelica anomala oil to obtain single volatile oily medicament.

No. 2 Experimental center, case No. 54, HASH, female, 65 years old. She had suffered from lassitude and hypodynamia, soreness and weakness in waists and knees, dizziness, tinnitus, short of breath, and frequency of micturition. Blood glucose was 11.59mmol/L, urinary glucose (U-GLU, ++++).

After the patient wore the shoes for 30 days, there was an outstanding improvement in lassitude and hypodynamia, shortness of breath, and frequency of micturition.Dizziness and tinnitus faded away. Blood glucose dropped to 5.74mmol/L, U-GLU (-). After 90 days, shortness of breath was recovered to the normal. Blood glucose turned to 5.30mmol/L.

### Example 2:

Add 3 parts by weight of borneol to 2 parts by weight of angelica anomala oil, 2 parts by weight of borneol to 2 parts by weight of angelica oil extract, 9 parts by weight of borneol to 5 parts by weight of clove oil, and mix them to obtain volatile oily medicament.

No. 2 Experimental center, case No. 58, LFHU, female, 65 years old. She had suffered fromasthenia, soreness and weakness in waists and knees, shortness of breath, dizziness and tinnitus. Blood glucose was 15.61mmol/L, U-GLU (+++), and daily injections of insulin was 42 units.

After the patient wore the shoes for 30 days, there was an outstanding improvement in dizziness and tinnitus. Blood glucose dropped to 10.15mmol/L, U-GLU (-). After 60 days, soreness and weakness in waists and knees were improved very markedly, dizziness and tinnitus were recovered to the normal. Blood glucose turned to 9.66mmol/L. After 90 days, shortness of breath was improved very markedly, soreness and weakness in waists and knees were recovered to the normal. Blood glucose dropped to 5.98mmol/L, daily injections of insulin was reduced to 36 units.

### Example 3:

(1) Weigh out raw materials: 6 parts by weight of ligusticum wallichii;
(2) Pick above raw material carefully, grind it into the powders and sieve the powders to obtain the raw medicinal powders;
(3) Add 3 parts by weight of borneol to the raw medicinal powders to obtain single raw medicinal powder.

No. 2 Experimental center, case No. 59, QZQI, male, 64 years old. He had suffered fromhypodynamia, soreness and weakness in waists and knees, dizziness and tinnitus. Blood glucose 15.98mmol/L, U-GLU (++++), daily injection of insulin: 40 units.

After the patient wore the shoes for 30 days, there was an outstanding improvement in soreness and weakness in waists and knees. Blood glucose dropped to 7.49mmol/L, U-GLU (-). After 60 days, dizziness and tinnitus were improved very markedly, soreness and weakness in waists and knees were recovered to the normal. Blood glucose dropped to 6.06mmol/L. After 90 days, dizziness and tinnitus were recovered to the normal. Blood glucose dropped to 6.0mmol/L, daily injections of insulin was reduced to 36 units.

### Example 4:

(1) Weigh out raw materials: 8 parts by weight of amomum cardamomum;
(2) Pick above raw material carefully, grind it into the powders and sieve the powders to obtain the raw medicinal powders;
(3) Add 9 parts by weight of borneol to the raw medicinal powders to obtain the single raw medicinal powders.

No. 2 Experimental center, case No. 63, DTSU, male, 53 years old. He had suffered from soreness and weakness in waists and knees, shortness of breath and dizziness. Blood glucose 16.34mmol/L, U-GLU (++++).

After the patient wore the shoes for 30 days, measured blood glucose dropped to 9.08mmol/L, U-GLU (-). After 60 days, soreness and weakness in waists and knees, and shortness of breath were improved very markedly, and dizziness disappeared. Blood glucose dropped to 8.48mmol/L. After 90 days, soreness and weakness in waists and knees were recovered to the normal. Blood glucose was reduced to 7.93mmol/L.

### Example 5:

(1) Weigh out raw materials: 4 parts by weight of angelica anomala and 5 parts by weight of clove;
(2) Pick carefully the above-mentioned raw materials, grind them into the powders and sieve the powders to obtain the respective raw medicinal powders.
(3) Add 4 parts by weight of borneol to each of the raw medicinal powders respectively and mix them to obtain the mixture of raw medicinal powders.

No.1 Experimental Center, case number 25, ZSLI, female, 62 years old. She had suffered from soreness and weakness in waists and knees, shortness of breath and dizziness. Blood glucose was 12.2mmol/L. After the patient wore the shoes for 30 days, soreness and weakness in waists and knees, shortness of breath and dizziness were improved markedly. Blood glucose dropped to 5.0mmol/L. After 60 days, blood glucose dropped to 5.2mmol/L. After 90 days, shortness of breath was improved very markedly, soreness and weakness in waists and knees and dizziness were recovered to the normal. Blood glucose dropped to 4.4mmol/L.

### (2) Coronary heart disease:

Conclusions to the studies by Inner Mongolia International Mongolian Hospital and Affiliated Hospital of Inner Mongolia Medical University:
In two hospitals 50 cases of patients of coronary heart disease were observed for 90 days individually, the effects are significant. Adverse events did not occur.

The body self-healing and recuperating shoes have significant effect on improving symptoms such as chest pain, chest distress, shortness of breath, palpitation, bradycardia, soreness and weakness in waists and knees.

The total effective rate was 94%, obvious effective rate 19%, effective rate 75%, ineffective rate 6%, cure-remarkable-effectiveness rate(clinical control +obvious effect) 19 %.

### ② Typical clinical cases:

### Example 1:

Add 5 parts by weight of borneol into 3 parts by weight of clove oil to obtain a single volatile oily medicament.

No.1 Experimental Center, case number 31, DSFE, male, 60 years old. He had suffered from chest distress, shortness of breath, heart palpitation, soreness and weakness in waists and knees for five years. After the patient wore the shoes for 30 days, shortness of breath was improved very markedly. After 60 days, chest pain, chest distress, soreness and weakness in waists and knees were improved very markedly. After 90 days, shortness of breath, palpitation, soreness and weakness in waists and knees were recovered to the normal.

### Example 2:

Add three parts by weight of borneol into 2 parts by weight of clove oil extract to obtain a single volatile oil medicament.

No.1 Experimental Center, case number 11, XYYZ, female, 53 years old. She had suffered from chest pain, chest distress, shortness of breath, palpitation, soreness and weakness in waists and knees for 2 years. After the patient wore the shoes for 30 days, palpitationwas improved very markedly, soreness and weakness in waists and knees turned better. After 60 days, chest pain, chest distress, shortness of breath, palpitation, soreness and weakness in waists and knees were improved very markedly. After 90 days, chest pain was improved very markedly, chest distress, shortness of breath, heart palpitation, soreness and weakness in waists and knees were recovered to the normal.

### Example 3:

(1) Weigh out raw materials: 8 parts by weight of amomum cardamomum;
(2) Pick above raw material carefully, grind it into the powders and sieve the powders to obtain the raw medicinal powders;
(3) Add 9 parts by weight of borneol into the raw medicinal powdersto obtain the single raw medicinal powders.

No. 2 Experimental center, case No. 75, AYQI, female, 46 years old. She had suffered from chest pain, chest distress, shortness of breath, heart palpitation, soreness and weakness in waists and knees for 1 and a half years. After the patient wore the shoes for 30 days, shortness of breath, soreness and weakness in waists and knees were recovered to the normal. After 60 days chest distress and palpitation return to normal.

### Example 4:

(1) Weigh out raw materials: 6 parts by weight of ligusticum wallichii;
(2) Pick above raw material carefully, grind it into the powders and sieve the powders to obtain the raw medicinal powders;
(3) Add 8 parts by weight of borneol into the raw medicinal powders to obtain the single raw medicinal powders.

No. 2 Experimental center, case No. 71, SCXI, female, 61 years old. She had suffered from chest distress, shortness of breath, palpitation and lassitude and hypodynamia for three years. After the patient wore the shoes for 30 days, lassitude and hypodynamia disappeared. After 60 days, shortness of breath was recovered to the normal. After 90 days, palpitationwas recovered to the normal.

### Example 5:

(1) Weigh out raw materials: weigh out 1 part by weight of ligusticum wallichii, 2 parts by weight of abutilon pepper, 2 parts by weight of amomum cardamom, 4 parts by weight of Angelica anomala and 1 part by weight of clove;
(2) Pick carefully each of the above-mentioned raw materials, grind them into the powders and sieve the powders to obtain the respective raw medicinal powders.
(3) Add 2 parts by weight of borneol into each of the raw medicinal powders respectively to obtain respective single raw medicinal powders;
(4) Soak above single raw medicinal powders respectively in glacial acetic acid, and then extract the powders individually by diacolation to obtain extracted stock solutions of single raw medicinal powders, and then mix the stock solutions of single raw medicinal powders with each other to obtain the mixture of stock solutions.

No. 2 Experimental center, case No. 65, SBHU, male, 64 years old. He had suffered from chest distress, shortness of breath, palpitation and hypodynamia for five years. After the patient wore the shoes for 30 days, chest distress was improved very markedly. After 60 days, shortness of breath was recovered to the normal. After 90 days, palpitation and hypodynamia disappeared.

### (3) Hypertension:

Conclusions to the clinical studies by Inner Mongolia International Mongolian Hospital and Affiliated Hospital of Inner Mongolia Medical University:
In two hospitals 50 cases of hypertension patients were observed for 90 days individually, the effects are significant. Adverse events did not occur.

The body self-healing and recuperating shoes have a significant improvement on symptoms such as headache, dizziness, palpitation, lassitude, hypodynamia, insomnia as well as soreness and weakness in waists and knees.

The total effective rate was 98%, obvious effective rate 46%, effective rate 52%, and ineffective rate 2%.

### ① Typical clinical cases:

### Example 1:

Add 5 parts by weight of borneol into 2 parts by weight of the Angelica anomala oil to obtain single volatile oily medicament.

No. 2 Experimental center, case No. 67, YECH, male, 65 years old. He had suffered from dizziness, headache, palpitation and insomnia for six years. Blood pressure was 150/100mmHg. After the patient wore the shoes for 30 days, dizziness was improved very markedly. Blood pressure was 140/90mmHg. After 60 days, headache and insomnia disappeared. Blood pressure was135/95mmHg. After 90 days, palpitationwas recovered to the normal. Blood pressure was 130/85mmHg.

### Example 2:

Add 5 parts by weight of borneol into 2 parts by weight of Angelica anomala oil, 4 parts by weight of borneol into 7 parts by weight of clove oil extract, and mix the materials to obtain volatile oily medicament.

No. 2 Experimental center, case No. 91, GYSH, male, 53 years old, doctor. He had suffered from feeling of dizziness, headaches, palpitation, soreness and weakness in waists and knees for five years. Blood pressure was150/100 mmHg. After the patient wore the shoes for 30 days, headache disappeared, soreness and weakness in waists and knees were recovered to the normal. Blood pressure was140/95mmHg. After 60 days, palpitation disappeared. After 90 days, dizziness disappeared. Blood pressure was 140/90mmHg.

### Example 3:

(1) Weigh out raw materials: 6 parts by weight of amomum cardamomum;
(2) Pick above raw material carefully, grind it into the powders and sieve the powders to obtain the raw medicinal powders;
(3) Add 9 parts by weight of borneol into the raw medicinal powders to obtain the single raw medicinal powders.

No. 2 Experimental center, case number 100, DTDI, male, 48 years old. He had suffered from headache, heart palpitation, insomnia, soreness and weakness in waists and knees for three years. Blood pressure was160/100mmHg. After the patient wore the shoes for 30 days, palpitation disappeared. After 60 days, insomnia disappeared. Blood pressure was140/90mmHg. After 90 days, headache disappeared; waists and knees were recovered to the normal. Blood pressure was 140/80mmHg.

### Example 4:

(1) Weigh out raw materials: 5 parts by weight of ligusticum wallichii;
(2) Pick above raw material carefully, grind it into the powders and sieve the powder to obtain the raw medicinal powders;
(3) Add 8 parts by weight of borneol into the raw medicinal powders, to obtain the single raw medicinal powders.

No. 2 Experimental center, case No. 80, QLLI, female, 51 years old. She had suffered from dizziness, headache, palpitation and insomnia for 1 year. Blood pressure was160/100mmHg. After the patient wore the shoes for 30 days, insomnia disappeared. Blood pressure was150/100mmHg. After 60 days, palpitation disappeared. Blood pressure was150/90mmHg. After 90 days, dizziness and headache disappeared. Blood pressure was 140/90mmHg.

### Example 5:

(1) Weigh out raw materials: 2 parts by weight of ligusticum wallichii, 3 parts by weight of abutilon pepper and 2 parts by weight of clove;
(2) Pick carefully each of the above-mentioned raw materials, grind it into the powders and sieve the powders to obtain the respective raw medicinal powders.
(3) Add 4 parts by weight of borneol into each of the kinds of the raw medicinal powders to obtain single raw medicinal powders.
(4) Soak above single raw medicinal powders respectively in glacial acetic acid, and then extract the powders individually by diacolation to obtain extracted stock solutions of single raw medicinal powders, and then mix the stock solutions of single raw medicinal powders with each other to obtain the mixture of stock solutions.

No.1 Experimental Center, case number 22, MYLI, male, 41 years old. He had suffered from dizziness, headache, palpitation and insomnia for 2 years. Blood pressure was 140/100mmHg. After the patient wore the shoes for 30 days, Blood pressure was120/80mmHg. After 60 days, headache, palpitation and insomnia disappeared. Blood pressure was 120/80mmHg.

### Example 6:

(1) Weigh out raw materials: 4 parts by weight of abutilon pepper, 2 parts by weight of amomum cardamom and 8 parts by weight of clove;
(2) Pick carefully each of the above-mentioned raw materials, grind it into the powders and sieve the powders to obtain the respective raw medicinal powders.
(3) Add 2 parts by weight of borneol into each of the raw medicinal powders respectively to obtain single raw medicinal powders;
(4) Soak above single raw medicinal powders respectively in glacial acetic acid, and then extract the powders individually by diacolation to obtain extracted stock solutions of single raw medicinal powders, and then mix the stock solutions of single raw medicinal powders with each other to obtain the mixture of stock solutions.

No. 2 Experimental center, case No. 85, MADA, male, 48 years old. He had suffered from feeling of dizziness, headache, palpitation and insomnia for three years. Blood pressure was145/95mmHg. After the patient wore the shoes for 30 days, insomnia disappeared. Blood pressure was 145/90mmHg. After 60 days, dizziness disappeared. Blood pressure was135/90mmHg. After 90 days, headache and palpitation disappeared. Blood pressure was 125/80mmHg.

### Example 7:

(1) Weigh out raw materials: 2 parts by weight of ligusticum wallichii, 4 parts by weight of abutilon pepper, 5 parts by weight of amomum cardamom, 8 parts by weight of angelica anomala and 4 parts by weight of clove;
(2) Pick carefully each of the above-mentioned raw materials, grind it into the powders and sieve the powders to obtain the respective raw medicinal powders.
(3) Add 7 parts by weight of borneol into each of the raw medicinal powders respectively, to obtain single raw medicinal powders;
(4) Soak above single raw medicinal powders respectively in glacial acetic acid, and then extract the powders individually by diacolation to obtain extracted stock solutions of single raw medicinal powders, and then mix the stock solutions of single raw medicinal powders with each other to obtain the mixture of stock solutions.

No. 2 Experimental center, case No. 92, NLGE, male, 46 years old. He had suffered from feeling of dizziness, headache, palpitation, lassitude and hypodynamia for 2 years. Blood pressure was145/95mmHg. After the patient wore the shoes for 30 days, dizziness disappeared. Blood pressure was140/90mmHg. After 60 days, palpitation, lassitude and hypodynamiawere recovered to the normal. Blood pressure was135/80mmHg. After 90 days, headache disappeared. Blood pressure was125/80mmHg.

### (4) Sequelae of cerebral infarction, stroke:

① Conclusions to the clinical studies by Inner Mongolia International Mongolian Hospital and Affiliated Hospital of Inner Mongolia Medical University:
   In the two hospitals 50 cases of sequelae of cerebral infarction and stroke patients were observed for 90 days individually, the effects are significant. Adverse events did not occur. The shoes being proposed in this invention can significantly improve symptoms such as hemiplegia, loss or complete loss of senses, deviation of mouth and tongue, speech difficulties, shortness of breath and so on. Adverse events did not occur. The cure rate3%, obvious effective rate 16%, effective rate 71%, ineffective rate 10%, cure-remarkable-effectiveness rate(cure +obvious effect) 19%. The total effective rate in the clinical test was 92%.
② Typical clinical cases:

### Example 1:

Add 2 parts by weight of borneol into 3 parts by weight of clove oil to obtain a single volatile oily medicament.

No. 2 Experimental center, case No. 93, MSKA, male, 63 years old. He had suffered from hemiplegia, numbness in the left limb, limb hypoesthesia, deviation of mouth and tongue, and mental fatigue for six months. After the patient wore the shoes for 30 days, sensory function of limbs was recovered to the normal. After 60 days, hemiplegia and numbness in the left limbs were recovered to the normal and the patient could take care of himself. After 90 days, deviation of mouth and tongue and mental fatigue were recovered to the normal.

### Example 2:

Add 7 parts by weight of borneol into 3 parts by weight of clove oil to obtain single volatile oily medicament.

No. 2 Experimental center, case No. 59, ZQSH, male, 53 years old. He had suffered from hemiplegia, limb hypoesthesia, and mental fatigue for eight months. After the patient wore the shoes for 30 days, mental fatigue was recovered to the normal. After 60 days, sensory function of limbs was recovered to the normal. After 90 days, hemiplegia, mental fatigue, shortness of breath and hypodynamia are removed.

### Example 3:

Add 5 parts by weight of borneol into 3 parts by weight of clove oil, 6 parts by weight of borneol into 2 parts by weight of angelica anomala oil, mix to obtain a mixed volatile oily medicament.

No. 2 Experimental center, case No. 53, LJXU, male, 60 years old. He had suffered from hemiplegia, limb hypoesthesia, tongue and mouth askew, shortness of breath and hypodynamia for four years. After the patient wore the shoes for 30 days, shortness of breath and hypodynamiawere improved very markedly. After 60 days, limb hypoesthesia, mental fatigue, shortness of breath and hypodynamiawere recovered to the normal. After 90 days, hemiplegia, tongue and mouth askew were recovered to the normal.

### Example 4:

(1) Weigh out raw materials: 8 parts by weight of ligusticum wallichii;
(2) Pick above raw material carefully, grind it into the powders and sieve the powders to obtain the raw medicinal powders;
(3) Add 9 parts by weight of borneol into the raw medicinal powders to obtain the single raw medicinal powders.

No. 2 Experimental center, case No. 58, WXHO, male, 55 years old. He had suffered from hemiplegia, limb hypoesthesia, tongue and mouth askew and shortness of breath for six years. After the patient wore the shoes for 30 days, limb hypoesthesiawas recovered to the normal, shortness of breath was improved very markedly. After 60 days, shortness of breath was recovered to the normal. After 90 days, deviation of mouth and tongue was recovered to the normal.

### Example 5:

(1) Weigh out raw materials: 6 parts by weight of ligusticum wallichii;
(2) Pick above raw material carefully, grind it into the powders and sieve the powders to obtain the raw medicinal powders;
(3) Add 8 parts by weight of borneol into the raw medicinal powders to obtain the single raw medicinal powders.

No. 2 Experimental center, case No. 52, CWBA, male, 58 years old. He had suffered from hemiplegia, limb hypoesthesia, shortness of breath, hypodynamia as well as spontaneous perspiration for four years. After the patient wore the shoes for 30 days, spontaneous perspiration was improved. After 60 days, limb hypoesthesiawas recovered to the normal. After 90 days, shortness of breath, hypodynamia, and spontaneous perspiration was recovered to the normal.

### Example 6:

(1) Weigh out raw materials: weigh out 5 parts by weight of abutilon pepper;
(2) Pick above raw material carefully, grind it into the powders and sieve the powders to obtain the raw medicinal powders;
(3) Add 8 parts by weight of borneol into the raw medicinal powders to obtain the single raw medicinal powders;
(4) Soak above single raw medicinal powders respectively in glacial acetic acid, and then extract the powders individually by diacolation to obtain extracted stock solutions of single raw medicinal powders, and then mix the stock solutions of single raw medicinal powders with each other to obtain the mixture of stock solutions.

No.1 Experimental Center, case No. 1, NYXI, female, 46 years old. She had suffered from hemiplegia, limb hypoesthesia, tongue and mouth askew and shortness of breath for one and a half years. After the patient wore the shoes for 30 days, tongue and mouth askew was recovered to the normal. After 60 days, limb hypoesthesiawas improved very markedly. After 90 days, shortness of breath was recovered to the normal, and hemiplegia was improved very markedly.

### Example 7:

(1) Weigh out raw materials: weigh out 4 parts by weight of angelica anomala and 4 parts by weight of clove;
(2) Pick carefully each of the above-mentioned raw materials, grind it into the powders and sieve the powders to obtain the respective raw medicinal powders.
(3) Add 8 parts by weight of borneol into each one kind of raw medicinal powders to obtain single raw medicinal powders;
(4) Soak above single raw medicinal powders respectively in glacial acetic acid, and then extract the powders individually by diacolation to obtain extracted stock solutions of single raw medicinal powders, and then mix the stock solutions of single raw medicinal powders with each other to obtain the mixture of stock solutions.

No.1 Experimental Center, case No. 33, TRBA, male, 62 years old. He had suffered from hemiplegia, limb hypoesthesia as well as tongue and mouth askew for 2 years. After the patient wore the shoes for 60 days, tongue and mouth askew was recovered to the normal, limb hypoesthesiawas improved very markedly. After 90 days, hemiplegia was improved very markedly.

### Example 8:

(1) Weigh out raw materials: 1 part by weight of ligusticum wallichii and 1 part by weight of clove;
(2) Pick carefully each of the above-mentioned raw materials, grind it into the powders and sieve the powders to obtain the respective raw medicinal powders.
(3) Add 2 parts by weight of borneol into each one kind of raw medicinal powders to obtain single raw medicinal powders;
(4) Soak above single raw medicinal powders respectively in glacial acetic acid, and then extract the powders individually by diacolation to obtain extracted stock solutions of single raw medicinal powders, and then mix the stock solutions of single raw medicinal powders with each other to obtain the mixture of stock solutions.

No.1 Experimental Center, case No. 39, WDPE, male, 60 years old. He had suffered from hemiplegia, limb hypoesthesia, tongue and mouth askew, shortness of breath as well as hypodynamia for six months. After the patient wore the shoes for 60 days, tongue and mouth askew was recovered to the normal, hemiplegia and limb hypoesthesiawere improved very markedly. After 90 days, shortness of breath and hypodynamiawere recovered to the normal.

### Example 9:

(1) Weigh out raw materials: 1 part by weight of ligusticum wallichii and 2 parts by weight of abutilon pepper;
(2) Pick carefully each of the above-mentioned raw materials, grind them into the powders and sieve the powders to obtain the respective raw medicinal powders.
(3) Add 8 parts by weight borneol into each of raw medicinal powders to obtain single raw medicinal powders;
(4) Soak above single raw medicinal powders respectively in glacial acetic acid, and then extract the powders individually by diacolation to obtain extracted stock solutions of single raw medicinal powders, and then mix the stock solutions of single raw medicinal powders with each other to obtain the mixture of stock solutions.

No.1 Experimental Center, case No. 46, LILI, female 47 years old. She had suffered from hemiplegia, sensory loss, mouth askew, shortness of breath and fatigue for 2 years. After the patient wore the shoes for 60 days, mouth askew, shortness of breath and fatigue were recovered to the normal, hemiplegia was improved very markedly. After 90 days, limb hypoesthesia was improved very markedly.

### (5) Cervical spondylosis:

① Conclusions to the studies by Inner Mongolia International Mongolian Hospital and Affiliated Hospital of Inner Mongolia Medical University: in the two hospitals 50 cases of cervical spondylosis patients were observed for 90 days individually, the effect is significant. Adverse events did not occur. The body self-healing and recuperating shoes have effects of significantly improving symptoms such as neck rigidity and feeling of pain, dizziness, headache, numbness in the limbs, palpitation and insomnia. Clinical cure rate was 6%, obvious effective rate 35%, effective rate 55%, ineffective rate 4%, and clinical total effective rate 96%.
②Typical clinical cases:

### Example 1:

Add 5 parts by weight of borneol into 3 parts by weight of clove oil extract to obtain a single volatile oily medicament.

No. 2 Experimental center, case No. 52, ZYXI, male, 42 years old. He had suffered from neck pain, numbness of limbs, dizziness and tinnitus. After 30 days of the patient wore the shoes, dizziness disappeared, tinnitus was improved very markedly. After 60 days, neck pain and numbness of limbs were recovered to the normal. Tinnitus was recovered to the normal after 90 days.

### Example 2:

Weigh out three parts by weight of borneol into 7 parts by weight of the angelica anomala oil to obtain a single volatile oily medicament.

No. 2 Experimental center, case No. 51, BAYU, male, 48 years old. He had suffered from pain in the neck, headaches, numbness of limbs and tinnitus. After the patient wore the shoes for 30 days, headache, and numbness of limbs were recovered to the normal. After 60 days, tinnitus was recovered to the normal. After 90 days, neck pain was recovered to the normal.

### Example 3:

Add three parts by weight of borneol into 7 parts by weight of angelica anomala oil, 3 parts by weight of borneol into 7 parts by weight of an extract of angelica oil, mix the materials with each other to obtain mixed volatile oily medicament.

No. 2 Experimental center, 87 cases, ZHYO, male, 51 years old. He had suffered fromlumbago, dizziness, headaches, numbness of limbs, palpitation and insomnia for 2 years. After the patient wore the shoes for 60 days, numbness of limbs was improved very markedly; insomnia was recovered to the normal. After 90 days, neck pain and headache were recovered to the normal.

### Example 4:

(1) Weigh out raw materials: 5 parts by weight of amomum cardamomum;
(2) Pick above raw material carefully, grind it into the powders and sieve the powders to obtain the raw medicinal powders;
(3) Add 9 parts by weight of borneol to the raw medicinal powders to obtain the single raw medicinal powders.

No. 2 Experimental center, case No. 56, YYSH, male, 38 years old. He had suffered from neck pain, headaches, numbness in limbs and insomnia for three years. After the patient wore the shoes for 30 days, neck pain, headaches and insomnia were improved very markedly. After 60 days, headache and numbness in limbs were recovered to the normal. After 90 days, insomnia was recovered to the normal.

### Example 5:

(1) Weigh out the raw materials: 5 parts by weight of ligusticum wallichii;
(2) Pick above raw material carefully, grind it into the powders and sieve the powders to obtain the raw medicinal powders;
(3) Add 9 parts by weight of borneol to the raw medicinal powders to obtain the single raw medicinal powders.

No. 2 Experimental center, case No. 60, GYLI, male, 50 years old. He had suffered from neck pain, dizziness, numbness in the limbs and insomnia for 2 years. After the patient wore the shoes for 30 days, numbness of limbs and insomnia were improved very markedly. After 60 days, neck pain was improved very markedly; dizziness and insomnia were recovered to the normal. After 90 days, numbness in limbs was recovered to the normal.

### Example 6:

(1) Weigh out the raw materials: 3 parts by weight of abutilon pepper;
(2) Pick above raw material carefully, grind it into the powders and sieve the powders to obtain the raw medicinal powders;
(3) Add 9 parts by weight of borneol into the raw medicinal powders to obtain the single raw medicinal powders.

No.1 Experimental Center, Case No. 42 CALI, male, 35 years old. He had suffered from neck pain, dizziness, headache, numbness in the limbs, palpitation and insomnia for 2 years. After the patient wore the shoes for 60 days, neck pain, headache, dizziness, numbness in the limbs, palpitation and insomnia were improved very markedly. After 90 days, neck pain, headache, dizziness, numbness in the limbs, palpitation and insomnia were recovered to the normal.

### Example 7:

(1) Weigh out raw materials: 6 parts by weight of the ligusticum wallichii, 2 parts by weight of abutilon pepper, 2 parts by weight of amomum cardamomum, 4 parts by weight of angelica anomala;
(2) Pick carefully each of the above-mentioned raw materials, grind them into the powders and sieve the powders to obtain the respective raw medicinal powders.
(3) Add 8 parts by weight of borneol into respective raw medicinal powders, and mix them to obtain the mixed raw medicinal powders.

No.1 Experimental Center, case number 27, HAGU, male, 47 years old. He had suffered from neck pain, dizziness, headache, numbness in the limbs and insomnia for six years. After the patient wore the shoes for 60 days, neck pain, dizziness, headache, numbness in the limbs and insomnia were improved very markedly. After 90 days, neck pain, dizziness, headache, numbness in the limbs and insomnia were recovered to the normal.

### Example 8:

(1) Weigh out raw materials: 1 part by weight of ligusticum wallichii, 2 parts by weight of abutilon pepper, 2 parts by weight of amomum cardamomum and 4 parts by weight of angelica anomala;
(2) Pick carefully each of the above-mentioned raw materials, grind them into the powders and sieve the powders to obtain the respective raw medicinal powders.
(3) Add 3 parts by weight of borneol into each of raw medicinal powders to obtain the single raw medicinal powders;
(4) Soak above single raw medicinal powders respectively in glacial acetic acid, and then extract the powders individually by diacolation to obtain extracted stock solutions of single raw medicinal powders, and then mix the stock solutions of single raw medicinal powders with each other to obtain the mixture of stock solutions.

No.1 Experimental Center, case number 17, QIYA, female, 47 years old. She had suffered from neck pain, dizziness, headache, numbness in the limbs, insomnia and tinnitus for five years. After the patient wore the shoes for 60 days, neck pain, dizziness, headache, numbness in the limbs, insomnia and tinnitus were improved very markedly. After 90 days, neck pain, dizziness, headache, numbness in the limbs, insomnia and tinnitus were recovered to the normal.

### Example 9:

(1) Weigh out raw materials: 2 parts by weight of abutilon pepper, 4 parts by weight of angelica anomala and 1 part by weight of clove;
(2) Pick carefully each of the above-mentioned raw materials, grind them into the powders and sieve the powders to obtain the respective raw medicinal powders.
(3) Add 6 parts by weight of borneol into each of raw medicinal powders respectively to obtain the single raw medicinal powders;
(4) Soak above single raw medicinal powders respectively in glacial acetic acid, and then extract the powders individually by diacolation to obtain extracted stock solutions of single raw medicinal powders, and then mix the stock solutions of single raw medicinal powders with each other to obtain the mixture of stock solutions.

No.1 Experimental Center, case number 05, DOYU, male, 35 years old. He had suffered from neck pain, dizziness, headache, numbness in the limbs and insomnia for seven years. After the patient wore the shoes for 60 days, neck pain, headache and dizziness were improved very markedly, numbness in the limbs and insomnia were recovered to the normal. After 90 days, neck pain, dizziness and headache were recovered to the normal.

### Example 10:

(1) Weigh out raw materials: 1 part by weight of ligusticum wallichii, 2 parts by weight of green hemp pepper, 2 parts by weight of amomum cardamomum, 4 parts by weight of angelica and 1 part by weight of clove;
(2) Pick carefully each of the above-mentioned raw materials, grind them into the powders and sieve the powders to obtain the respective raw medicinal powders.
(3) Add 2 parts by weight of borneol into each of raw medicinal powders to obtain the single raw medicinal powders;
(4) Soak above single raw medicinal powders respectively in glacial acetic acid, and then extract the powders individually by diacolation to obtain extracted stock solutions of single raw medicinal powders, and then mix the stock solutions of single raw medicinal powders with each other to obtain the mixture of stock solutions.

No.1 Experimental Center, case number 26, ZQPI, female, 35 years old. She had suffered from neck pain, headache, dizziness, numbness in the limbs, and insomnia for five years. After the patient wore the shoes for 60 days, neck pain, headache, dizziness, numbness in the limbs, insomnia and tinnitus were improved very markedly. After 90 days, neck pain, headache, dizziness, numbness in the limbs, insomnia and tinnitus were recovered to the normal.

### (6) lumbar spondylosis:

① Conclusions to the studies by Inner Mongolia International Mongolian Hospital and Affiliated Hospital of Inner Mongolia Medical University: in two hospitals 50 cases of lumbar spondylosis patients were observed for 90 days individually, the effects are significant. Adverse events did not occur. The body self-healing and recuperating shoes have significant effects on improving symptoms such as lumbago, affection of kidney by cold-dampness, soreness and weakness in waists and knees. The total effective rate was 92%, clinical curerate 0%, obvious effective rate 18%, effective rate 74%, ineffective rate 8%;
② Typical clinical cases:

### Example 1:

Add 5 parts by weight of borneol into 3 parts by weight of clove oil to obtain a single volatile oily medicament.

No. 2 Experimental center, case No. 84, SSYU, male, 69 years old. He had suffered fromlumbago, affection of kidney by cold-dampness, soreness and weakness in waists and knees for six years. After using the body self-healing and recuperating shoes for 30 days,aversion to cold, cold limbs and soreness and weakness in waists and knees were improved very markedly. After 60 days, aversion to cold, cold limbs, soreness and weakness in waists and knees and lumbagowere recovered to the normal. After 90 days, affection of kidney by cold-dampnesswas recovered to the normal.

### Example 2:

Add three parts by weight of borneol into 2 parts by weight of clove oil extract to obtain a single volatile oily medicament.

No. 2 Experimental center, case No. 98, HAYA, female, 46 years old. She had suffered fromlumbago, affection of kidney by cold-dampness as well as soreness and weakness in waists and knees for four years. After the patient wore the shoes for 30 days, soreness and weakness in waists and knees turned to normal. After 60 days, affection of kidney by cold-dampnessand lumbago were recovered to the normal.

### Example 3:

(1) Weigh out raw materials: 8 parts by weight of amomum cardamomum;
(2) Pick above raw material carefully, grind it into the powders and sieve the powders to obtain the raw medicinal powders;
(3) Add 9 parts by weight of borneol into the raw medicinal powders to obtain the single raw medicinal powders.

No. 2 Experimental center, 87 cases, ZHYO, male, 39 years old. He had suffered from lumbago, affection of kidney by cold-dampness, and soreness and weakness in waists and knees for 2 years. After the patient wore the shoes for 30 days soreness and weakness in waists and knees were improved very markedly. After 60 days, soreness and weakness in waists and knees were recovered to the normal. After 90 days, affection of kidney by cold-dampnessand lumbago were recovered to the normal.

### Example 4:

(1) Weigh out raw materials: 8 parts by weight of ligusticum wallichii;
(2) Pick above raw material carefully, grind it into the powders and sieve the powders to obtain the raw medicinal powders;
(3) Add 9 parts by weight of borneol into the raw medicinal powders to obtain the single raw medicinal powders.

No. 2 Experimental center, case No. 82, WWDO, male 48 years old. He had suffered fromlumbago, affection of kidney by cold-dampness as well as soreness and weakness in waists and knees. After the patient wore the shoes for 30 days, soreness and weakness in waists and knees were improved very markedly. After 60 days, affection of kidney by cold-dampnesswas recovered to the normal. After 90 days, aversion to cold and cold limbs were improved very markedly, lumbagoas well as soreness and weakness in waists and knees were recovered to the normal.

### Example 5:

(1) Weigh out raw materials: 8 parts by weight of angelica anomala;
(2) Pick above raw material carefully, grind it into the powders and sieve the powders to obtain the raw medicinal powders;
(3) Add 9 parts by weight of borneol into the raw medicinal powders to obtain the single raw medicinal powders.

No. 2 Experimental center, case No. 96, ZCYI, female, 61 years old. Shehad suffered fromlumbago, affection of kidney by cold-dampness, as well as soreness and weakness in waists and knees for five years. After the patient wore the shoes for 60 days, soreness and weakness in waists and knees were improved very markedly. After 90 days, affection of kidney by cold-dampness, soreness and weakness in waists and knees and lumbagowere recovered to the normal.

### Example 6:

(1) Weigh out raw materials: 6 parts by weight of ligusticum wallichii and 3 parts by weight of clove;
(2) Pick carefully each of the above-mentioned raw materials, grind them into the powders and sieve the powders to obtain the respective raw medicinal powders
(3) Add 5 parts by weight of borneol into respective raw medicinal powders, and mix them to obtain the mixture of raw medicinal powders.

No.1 Experimental Center, number of cases 14, DHLZ, female, 29 years old. She had suffered fromlumbago, affection of kidney by cold-dampness, and soreness and weakness in waists and knees for 1 year.

After the patient wore the shoes for 60 days, affection of kidney by cold-dampness as well as soreness and weakness in waists and knees were recovered to the normal, and the aggravation of lumbago in case of coldwas relieved very markedly.

After 90 days, the aggravation of lumbago in case of cold, affection of kidney by cold-dampness, soreness and weakness in waists and knees as well as lumbagowere recovered to the normal.

### Example 7:

(1) Weigh out raw materials: 2 parts by weight of abutilon pepper, 2 parts by weight of amomum cardamomum and 6 parts by weight of clove;
(2) Pick carefully each of the above-mentioned raw materials, grind it into the powders and sieve the powders to obtain the respective raw medicinal powders.
(3) Add 3 parts by weight of borneol into each of raw medicinal powders to obtain single raw medicinal powders;
(4) Soak above single raw medicinal powders respectively in glacial acetic acid, and then extract the powders individually by diacolation to obtain extracted stock solutions of single raw medicinal powders, and then mix the stock solutions of single raw medicinal powders with each other to obtain the mixture of stock solutions.

No.1 Experimental Center, case number 18, ZCHO, female, 50 years old.

Shehad suffered fromlumbago, affection of kidney by cold-dampness, the aggravation of lumbago in case of coldas well as soreness and weakness in waists and knees for 2 years.

After the patient wore the shoes for 60 days, affection of kidney by cold-dampness,the aggravation of lumbago in case of cold as well as soreness and weakness in waists and knees were improved very markedly.

After 90 days, affection of kidney by cold-dampness as well as soreness and weakness in waists and knees were recovered to the normal, and lumbagowas improved very markedly.

### Example 8:

(1) Weigh out raw materials: 4 parts by weight of root, 6 parts by weight of abutilon pepper, 2 parts by weight of amomum cardamomum, 4 parts by weight of angelica anomala and 9 parts by weight of clove;
(2) Pick carefully each of the above-mentioned raw materials, grind them into the powders and sieve the powders to obtain the respective raw medicinal powders.
(3) Add 5 parts by weight of borneol into each kind of raw materials to obtain the single raw medicinal powders;
(4) Soak above single raw medicinal powders respectively in glacial acetic acid, and then extract the powders individually by diacolation to obtain extracted stock solutions of single raw medicinal powders, and then mix the stock solutions of single raw medicinal powders with each other to obtain the mixture of stock solutions.

No.1 Experimental Center, case No. 39, WXDO, male, 39 years old. He had suffered fromlumbago, the aggravation of lumbago in case of cold, affection of kidney by cold-dampness, as well as soreness and weakness in waists and knees for 2 years.

After the patient wore the shoes for 60 days, lumbago, the aggravation of lumbago in case of cold, affection of kidney by cold-dampnessas well as soreness and weakness in waists and knees were improved very markedly.

After 90 days, the aggravation of lumbago in case of cold,affection of kidney by cold-dampness as well assoreness and weakness in waists and knees were recovered to the normal.

### (7) Periarthritis of shoulders:

① Conclusions to the studies by Inner Mongolia International Mongolian Hospital and Affiliated Hospital of Inner Mongolia Medical University: the clinical observation of two hospitals on 50 cases of patients of periarthritis of shoulders was carried out for 90 days, the clinical effect was significant. The symptoms of soreness and pain in shoulders, motion limitation of shoulders, dizziness, shortness of breath, palpitation and insomnia were improved significantly by means of wearing the body self-healing and recuperating shoes. Adverse events did not occur. Clinical cure rate was 13%, obvious effective rate was16%, effective rate was 67%. The total effective rate was 96%, cure-remarkable-effectiveness rate(cure +obvious effect) 29%, ineffective rate 4%.
②Typical clinical cases:

### Example 1:

2 parts by weight of borneol were added to 3 parts by weight of clove oil to obtain a single volatile oily medicament.

No. 2 Experimental center, case No. 80, LXFA, female, 59 years old. She had suffered from soreness and pain in shoulders, limited activity, dizziness, blurred vision, shortness of breath, palpitation and insomnia for 2 years. After the patient wore the shoes for 30 days, dizziness and shortness of breath were recovered to the normal. After 60 days, shoulder motion limitation, heart palpitation and insomnia were recovered to the normal. After 90 days, soreness and pain in shoulders and limited activitywere recovered to the normal.

### Example 2:

Add three parts by weight of borneol to 7 parts by weight of clove oil extract to obtain a single volatile oily medicament.

No. 2 Experimental center, case No. 76, LZJU, male, 40 years old. He had suffered from soreness and pain in shoulders, motion limitation of shoulders, shortness of breath for 1 year. After the patient wore the shoes for 30 days, shortness of breath was recovered to the normal. After 60 days, shoulder motion limitation was recovered to the normal. After 90 days, soreness and pain in shoulders were recovered to the normal.

### Example 3:

(1) Weigh out raw materials: 5 parts by weight of amomum cardamomum;
(2) Pick above raw material carefully, grind it into the powders and sieve the powders to obtain the raw medicinal powders;
(3) Add 9 parts by weight of borneol into the raw medicinal powders to obtain the single raw medicinal powders.

No. 2 Experimental center, case No. 54, MOPI, male, 43 years old. He had suffered from the pain in the shoulders, motion limitation, shortness of breath, palpitation and insomnia. After the patient wore the shoes for 30 days, shortness of breath, palpitation, and insomnia were recovered to the normal. After 60 days, the shoulder motion limitation was recovered to the normal.

### Example 4:

(1) Weigh out raw materials: 8 parts by weight of abutilon pepper;
(2) Pick above raw material carefully, grind it into the powders and sieve the powders to obtain the raw medicinal powders;
(3) Add 9 parts by weight of borneol into the raw medicinal powders to obtain the single raw medicinal powders.

No.1 Experimental Center, case No. 02, GXNI, male, 30 years old, a doctor. He had suffered from soreness and pain in shoulders, limited activity, palpitation and insomnia for 2 years. After the patient wore the shoes for 60 days, soreness and pain in shoulders, shoulder motion limitation, heart palpitation and insomnia were improved very markedly. After 90 days, soreness and pain in shoulders, shoulder motion limitation, palpitation and insomnia were recovered to the normal.

### Example 5:

(1) Weigh out raw materials: 6 parts by weight of clove;
(2) Pick above raw material carefully, grind it into the powders and sieve the powder sto obtain the raw medicinal powders;
(3) Add 4 parts by weight of borneol into the raw medicinal powders to obtain the single raw medicinal powders.

No.1 Experimental Center, case number 04, NYFE, male, 55 years old, a doctor. He had suffered from soreness and pain in shoulders, limited activity, dizziness, shortness of breath, palpitation and insomnia. After the patient wore the shoes for 60 days, soreness and pain in shoulders, limited activity, shortness of breath, dizziness, palpitation and insomnia were improved very markedly. After 90 days, soreness and pain in shoulders, limited activity, dizziness, shortness of breath, palpitation and insomnia were recovered to the normal.

### Example 6:

(1) Weigh out raw materials: 6 parts by weight of ligusticum wallichii and 5 parts by weight of clove;
(2) Pick carefully each of the above-mentioned raw materials, grind them into the powders and sieve the powders to obtain the respective raw medicinal powders.
(3) Add 4 parts by weight of the borneol into each of raw medicinal powders, and mix them to obtain the mixed raw medicinal powders.

No.1 Experimental Center, case number 16, QLXI, female, 32 years old. She had suffered from soreness and pain in shoulders, limited activity, dizziness, shortness of breath, palpitation and insomnia for three years. After the patient wore the shoes for 60 days, soreness and pain in shoulders, limited activity, shortness of breath, dizziness, palpitation and insomnia were improved very markedly. After 90 days, soreness and pain in shoulders, limited activity, dizziness, shortness of breath, palpitation and insomnia were recovered to the normal.

### Example 7:

1) Weigh out raw materials: 4 parts by weight of angelica anomala and 4 parts by weight of clove;
(2) Pick carefully each of the above-mentioned raw materials, grind them into the powders and sieve the powders to obtain the respective raw medicinal powders.
(3) Add 4 parts by weight of borneol into each of raw medicinal powders to obtain the single raw medicinal powders;
(4) Soak above single raw medicinal powders respectively in glacial acetic acid, and then extract the powders individually by diacolation to obtain extracted stock solutions of single raw medicinal powders, and then mix the stock solutions of single raw medicinal powders with each other to obtain the mixture of stock solutions.

No.1 Experimental Center, case number 18, YYQZ, female, 45 years old.

She had suffered from soreness and pain in shoulders, limited activity, dizziness, shortness of breath, palpitation and insomnia for 2 years.

After the patient wore the shoes for 60 days, soreness and pain in shoulders, limited activity, dizziness, shortness of breath, palpitation and insomnia were improved very markedly.

After 90 days, soreness and pain in shoulders, limited activity, dizziness, shortness of breath, palpitation and insomnia were recovered to the normal.

### Example 8:

(1) Weigh out raw materials: 2 parts by weight of ligusticum wallichii, 2 parts by weight of abutilon pepper, 5 parts by weight of amomum cardamomum, 4 parts by weight of Angelica anomala and 7 parts by weight of clove;
(2) Pick carefully each of the above-mentioned raw materials, grind them into the powders and sieve the powders to obtain the respective raw medicinal powders.
(3) Add 7 parts by weight borneol into each of raw medicinal powders to obtain the single raw medicinal powders;
(4) Soak above single raw medicinal powders respectively in glacial acetic acid, and then extract the powders individually by diacolation to obtain extracted stock solutions of single raw medicinal powders, and then mix the stock solutions of single raw medicinal powders with each other to obtain the mixture of stock solutions.

No.1 Experimental Center, case No. 33, SSQI, female, 62 years old. She had suffered from soreness and pain in shoulders, limited activity, dizziness, palpitation and insomnia for 2 years. After the patient wore the shoes for 60 days, soreness and pain in shoulders, limited activity, dizziness, shortness of breath, palpitation and insomnia were improved very markedly. After 90 days, soreness and pain in shoulders, limited activity, dizziness, shortness of breath, palpitation and insomnia were recovered to the normal.

Following are the embodiments of disease treatment with combinations of medicinal magnetic therapy:
Wherein the preparation processes of medicaments are as follows:
(1) Weigh out raw materials: 2 parts by weight of ligusticum wallichii, 2 parts by weight of abutilon pepper, 5 parts by weight of amomum cardamomum, 4 parts by weight of angelica anomala and 7 parts by weight of clove;
(2) Pick carefully each of the above-mentioned raw materials, grind them into the powders and sieve the powders to obtain the respective raw medicinal powders.
(3) Add 7 parts by weight of borneol into each of raw medicinal powders to obtain the single raw medicinal powders;
(4) Soak above single raw medicinal powders respectively in glacial acetic acid, and then extract the powders individually by diacolation to obtain extracted stock solutions of single raw medicinal powders, and then mix the stock solutions of single raw medicinal powders with each other to obtain the mixture of stock solutions.

Magnetic beads are equipped in the uppers, insole and back counter of the shoes in the body self-healing and recuperating shoes.

### Example 1:

Lin Yongqing, 81 years old, began to wear the shoes in 2010 who lives in Majiapu Road 33., Yangqiao in Fengtai District, Beijing. He had suffered from diabetes for more than 10 years. Blood glucose 19.0mmol/L, low limbs felt itch. On the fourth day of wearing shoes, there was no feeling of itch. After wearing shoes for three months, the patient totally stopped taking hypoglycemic drugs, and blood glucose dropped to 5.8. He had suffered from hypertension for 17 years, systolic blood pressure (SBP) was 200mmHg, and diastolic blood pressure (DBP) was over 100 mmHg. After the patient wore shoes for three months, SBP dropped to 130-120, DBP to 90-85.

Example 2: Yi Guangcheng, 65 years old, began to wear the shoes in 2010 who lives in Tonghu Avenue Tongzhou District, Beijing. He had suffered from diabetes and hypertension. Blood glucose was 9.6 mmol/L, U-GLU (+++), acetone body 2 (++), SBP 170mmHg, DBP 100 mmHg. After the patient wore the shoes for a month, U-GLU and ketone body were reduced to (-). After three months, blood glucose dropped to 5.0 mmol/L.After the patient wore the shoes for a month, SBP dropped to 135 mmHg, DBP to 90 mmHg. After three months, SBP dropped to 120 mmHg, DBP to 80 mmHg.

### Example 3:

Li Xiuzhi, 74 years old, began to wear the shoes in 2010 who lives in Building 8, Xiangyangli, Yanshan district, Beijing. He had suffered from diabetes and hypertension. Blood glucose was 20.0 mmol/L, and he had suffered from pain in legs, numbness in legs and hands, SBP 180mmHg, DBP 110 mmHg. After the patient wore the shoes for three months, blood glucose dropped to 6.0mmol/L, no numbness in legs and hands, SBP dropped to 140 mmHg, DBP to 90 mmHg. He had suffered fromlumbago before wearing the shoes and could not do housework, even bent over. After wearing the shoes, he didn't felt pain in waist; presently it is no problem for him either to stoop or to stand straight.

### Example 4:

Yang Chongli, 62 years old, began to wear the shoes in 1997 who lives in Shiqiao, Miyun county, Beijing. He had suffered from hypertension(SBP 160mmHg, DBP 110 mmHg) coronary heart disease, and myocardia ischemia. After the patient wore the shoes for three months, results of several ECG examinations were normal, and myocardia ischemia disappeared, and SBP dropped to 130-140 mmHg, DBP to 90-95 mmHg. Suffering from cerebral thrombosis hemiplegia for 3 years, he could not leave bed and only go to the toilet supported by two people. After wearing the shoes for six months, he could practice walking leaning on two crutches, and he was able to walk more than 100 meters.

### Example 5:

Shuxian He, 65 years old, began to wear the shoes in 1997 who lives in China Academy of Railway Sciences, Beijing. He had suffered from coronary heart disease, angina. After the patient wore the shoes for half a month or so, angina disappeared, several examinations of electrocardiogram showed normal. He also had suffered from cerebral complications, left hand felt numb and could not grip things. During walking the left leg swung circularly. After the patient wore the shoes for three months, the leg did not swing during walking, and his left hand also had strength to grip things. He also had suffered from sequel of lupus erythematosus, and was liable to have a low fever and epistaxis when the spring, even the fever continued until October 1 of the year, the highest fever was 38 degrees. No medicine or injection had effects. After wearing the shoes, the patient didn't get a fever until now.

### Example 6:

YANG Mingde, 71 years old, began to wear the shoes in 1996 who lives in the Jinshan county, Tinglin District, Shanghai. He had suffered from diabetes(blood glucose 15mmol/L), hypertension(SBP 180mmHg, DBP 100), stroke three times, and was hospitalized three times. After he wore the shoes for three months, blood glucose and blood pressure turned to normal, and have been normal for two years.

### Example 7:

Su Deqing, male, 69 years old, Meng Fu, female, 70 years old (a couple), began to wear the shoes since 2010 who lives in YongFu community, Wen'an town, Langfang city. Su Deqing had suffered from diabetes for more than 10 years,blood glucose 10-12mmol/L, and U-GLU +++.The main clinical manifestations were emaciation and a pale complexion. After the patient wore the shoes for three months, his blood glucose dropped to 6.0 mmol/L, U-GLU + and emaciation disappeared, and he had a high color. Meng Fu had suffered from hypertension(SBP 180mmHg, DBP 110 mmHg) and headache. After the patient wore the shoes for three months, SBP dropped to 120 mmHg, DBP to 80 mmHg, and she does not suffer from headache anymore. The patient could not fall asleep previously for many years. After wearing the shoes, Meng could fall asleep for at least 5-6 hours a night.

### Example 8:

Wan Qingrong, 77 years old, began to wear the shoes in 2009 who lives in Liyuan village, South Jianta town, Langfang city. He had suffered from diabetes. Blood glucose was 11-12mmol/L. After the patient wore the shoes for three months, his blood glucose dropped to 4.2-5.8 mmol/L. Wang also had suffered from hypertension.SBP was 180-190mmHgand DBP was 100-95 mmHg. After the patient wore the shoes for 3 months, SBP dropped to 120-125 mmHg, DBP dropped to 75-80-85 mmHg. Wang also had suffered from cervical spondylosis, lay in bed for three years, and could not move because of dizziness. After three months, the neck did not make a sound when he turned his neck, even if he shakes the head, he did not feel dizziness and blur version disappeared.

### Example 9:

Wang Lin, male, 75 years old, Gao Yuqin, female, 64 years old (a couple), began to wear the shoes in 2008 who lives in Xinhualou, Tangjia village, Tangshan city. Wang Lin had suffered from coronary heart disease and angina, was rescued once in 2003 and once in 2008.After leaving the hospital,he felt weakness in limbs and walked painstakingly. After the patient wore the shoes for three months, the symptoms disappeared, and angina did not recur any more. He can do simple manual labor now. Gao Yuqin had suffered from diabetes. Her blood glucose was 19.4mmol/L, and she injected 36-40 units insulin daily. After the patient wore the shoes for three months, her blood glucose dropped to 5.0-6.0 mmol/L, and it was not necessary for her to inject insulin any more. Previously her vision was so blurred that she did not see people clearly from dozens of meters away. After wearing the shoes, she can see very clearly now. She also had suffered from rheumatoid arthritis, walking lamely. After wearing the shoes for three months, she was not lame and did not feel pain in legs and feet either. She also had suffered from Meniere's syndrome,got ill once a week, and at least once a month. The main clinical manifestations were coma, tinnitus. She cannot talk, cannot open her eyes and move, and she often vomit so as to throw up gall water. Previously because there was no enough time for her to go to the toilet, her husband had to use a pot to take shit and urine for her on the bed. She has not had suffered from the Meniere's syndrome, headache, coma and emesia since she wore the shoes.

### Example 10:

Li Shugui, male, 71 years old, Dong Hezhen, female, 70 years old (a couple), began to wear the shoes in 2010 who live in Xingfu Road, Tangche district, Tangshan city. Li had suffered from hypertension(SBP 160mmHg, DBP 110 mmHg), dizziness and headache. After the patient wore the shoes for three months, SBP dropped to 125-130 mmHg, DBP to 80-85 mmHg, and he did not feel dizziness and headache anymore. He also had suffered from cronary heart disease, angina, and one pause between 3-5 heartbeats, which caused him very uncomfortable. He also had suffered from prostatitis, not finishing urination, urination weak and urodynia, and he urinated 4 times per night. After the patient wore the shoes for two months, intermittent heartbeat basically disappeared, and angina disappeared. He urinated only once per night, symptoms such as urodynia, urination weak and seeming urination disappeared. Dong Hezhen had suffered from diabetes(blood glucose was 18.2mmol/L), and he injected 48 units of insulin daily. After wearing the shoes for three months, he began to reduce insulin injection; a little bit reduction every day, finally a daily insulin injection was reduced to 16 units two times in the morning and evening of a day. Blood glucose dropped to 6.0 mmol/L. Dong also had suffered from coronary heart disease, angina, and got ill a few times a month. After wearing the shoes for three months, he did not suffer from angina. She also had suffered from hypertension for 20 years, although all kinds of antihypertensive drugs had been tried, SBP was still 160-170mmHg, and DBP was 110 mmHg. After the patient wore the shoes for three months, it is almost not necessary for him to take antihypertensive drugs;SBP was stabilized at 120-130 mmHg, DBP at 74-80 mmHg.

### Example 11:

Zhang Shuchun, 76 years old, began to wear the shoes in 2011 who lives in Fandali railway building, Tangshan city. Zhang had suffered from diabetes, and his blood glucose was 11.9mmol/L. After the patient wore the shoes for three months, his blood glucose dropped to 6.2mmol/L. He had suffered from the rotten legs, complications of diabetic for ages, outer sides of two legs had tinea nigra with lengths of more than 200 millimeters, and they were so terribly itching that he scratched them bleeding. After wearing the shoes for three months, he did not have itching, and he has not haditching until now. The patient also had suffered from hypertension for 20 years, used to control the disease by taking antihypertensive drugs, yet SBP was 180mmHg, DBP 100-110 mmHg. Zhang had suffered from dizziness and coma. After the patient wore the shoes for 3 months, high blood was stabilized at 110 mmHg, DBP at 80-85 mmHg.

### Example 12:

Geng Ying, 59 years old, begun to wear the shoes in 2010 who lives in Beijiadian First Street, Wuzhuangzi, Tangshan City. He had suffered from diabetes for 17 years(glucose 14.0mmol/L), itch all over the body, and labor pains in hands and feet, as well as blurred eyes. After the patient wore the shoes for 3 months, his blood glucose dropped to 5.6 mmol/L. Feeling of itch in the whole body as well as labor pains in fingers disappeared, he could see clearly. Geng also suffered from cerebral infarction, during walking the legs swung circularly and could not bend, when he walked, the legs were thrown out first and then were drown back with hips, with the posture of hands like carrying baskets and the hands could not move. After the patient wore the shoes for nearly four months, the hand could move freely and could lift anything, and during walking the leg did not swing circularly, legs and feet could be lifted, and legs could bend and walk. The patient also had suffered fromprostatic hypertrophy and urinated at least 5-6 times per night. After the patient wore the shoes for two months, urination at night returned to normal, once per night.

### Example 13:

Liu Cuihua, 60 years old, began to wear the shoes in 2011 who lives in the 10th New workers' dormitory, Xinxing Road, Zhaogezhuang, Tangshan city. Liu had suffered from diabetes. Blood glucose was 13-26mmol/L, and he injected 40 units of insulin per day. After the patient wore the shoes for two and a half months, his blood glucose dropped to 5.3-5.7 mmol/L, injection of insulin was not necessary any more. Previously he could not see clearly, after wearing the shoes, he could see clearly.

### Example 14:

Zhu Chenglan, 68 years old, began to wear the shoes in 2010 who lives in Nanfange village, Tangshan city. He had suffered from diabetes for 21 years. Blood glucose was 17.0-18.0mmol/L, or even to 22.0 mmol/L, U-GLU +++. After the patient wore the shoes for three months, his blood glucose dropped to 5.5 mmol/L, U-GLU + disappeared. Zhu also had suffered from coronary heart disease, intermittent heart beats, palpitation and shortness of breath. After three months, these symptoms disappeared. Zhu also had suffered from hypertension, SBP was 220mmHg, when he was in hospital, SBPwas 230mmHg, and DBPwas58-60 mmHg. Zhu could not walk owing to dizziness. After the patient wore the shoes for two months, SBP dropped to 130 mmHg, DBP to 80 mmHg. He felt no more dizziness. Zhu also had suffered from nephritis, urinary protein +++, he had edema on the eyes and legs. After the patient wore the shoes for three months, urinary protein gradually disappeared, and he did not have edema on the eyes and legs.

### Example 15:

Hu Xilin, 71 years old, began to wear the shoes in 2008 who lives in No. 551 family's dormitory area, Headquarters of the Fifth Farm, Tangshan city. He had suffered from diabetes. Blood glucose was 8.0mmol/L and more, U-GLU ++. After the patient wore the shoes for 2 months, his blood glucose dropped to more than 5.0mmol/L, and maintained it until now. Presently Zhu takes basically no hypoglycemic drugs without causing any problem, U-GLU + disappeared. Previously Hu had suffered from skin itching, After the patient wore the shoes for one month, skin itching disappeared. Hu also had suffered from cardiac arrhythmia, premature beat and hypertension. Previously SBP was 160-170mmHg, DBP 90-100mmHg. After the patient wore the shoes for two months, the ECG examination results turned to normal. SBP dropped to 135mmHg, DBP to 70-80mmHg.

### Example 16:

Zhang Wenxiu, 63 years old, began to wear the shoes in 2010 who lives in Sanyi village, Tangshan city. He had suffered from diabetes for 23 years. Blood glucose was 9.7mmol/L, and he had complications of numb hands and feet. After the patient wore the shoes for more than two months, his blood glucose dropped to 6.0mmol/L. After the patient wore the shoes for more than 20 days, numbness in hands and feet was improved very markedly. The patient also had suffered from coronary heart disease and angina. After the patient wore the shoes for two months, coronary heart disease and angina was cured, The patient did not feel pain and nervous. The patient also had suffered from hypertension, SBP 170mmHg, DBP 87 mmHg. After the patient wore the shoes for two months, SBP dropped to 120 mmHg, DBP to 80 mmHg. The patient also suffered from sciatic nerve pain, had to rest for a while after walking for a while, or else could not persist in walking. After wearing the shoes, now the patient does not feel pain anymore.

### Example 17:

Ma Xiaolan, 63 years old, began to wear the shoes in 2011 who lives in West Yuehenan street, Nüzhizhai Township, Tangshan city. He had suffered from diabetes. Blood glucose was 13.8mmol/L. After the patient wore the shoes for 5 months, his blood glucose dropped to 6.2 mmol/L. Ma also had suffered from hypertension. SBP was 220mmHg, and DBP was 140 mmHg. After the patient wore the shoes for five months, SBP dropped to 140 mmHg, DBP to 90mmHg. Ma also had suffered from lumbar disc herniation, could not walk, only stayed in bed. After wearing the shoes, now Ma can ride a tricycle.

Example 18: Li shuyuan, 76 years, began to wear the shoes in 2011 who lives in Nanchanglou, Tangshan city. Li had suffered from diabetes for 4 years. Blood glucose was about 7-9mmol/L. After the patient wore the shoes for two and a half months, his blood glucose dropped to about 6.5 mmol/L. Li also suffered from hypertension. SBP was 160mmHg, and DBP was 90 mmHg. After the patient wore the shoes for two and a half months, SBP dropped to 140 mmHg, DBP to 80mmHg. Li had wounded his legs; his legs felt always pain, walking with difficulties. After the patient wore the shoes for more than two months, pain was relieved, now Li walked easily.

### Example 19:

Chen caizhen, 60 years old, began to wear the shoes in 2010 who lives in Guye district, Nangongfang community, Tangshan city. Chen had suffered from diabetes. Blood glucose 19.0-18.0mmol/L, and U-GLU ++++. After the patient wore the shoes for three months, her blood glucose dropped to over 8.0 mmol/L, and U-GLU + disappeared. Chen also suffered from hypertension, SBP 200-190mmHg, DBP 110-120 mmHg. After the patient wore the shoes, her Blood pressure turned to normal, 80/120 mmHg presently. She does not take antihypertensive drugs anymore now.

### Example 20:

Yang Jiang, 66 years old, began to wear the shoes in 2011 who lives in No. 3, 9th Zhouguantun Street, Tangshan. He had suffered from diabetes(blood glucose13.9 mmol/L), blurred vision, and he always felt drowsy no matter how much he slept, the whole body felt soreness and hypodynamia. After the patient wore the shoes for more than one month, the vision was not blurred, he did not feel sleepy anymore, as he walk he did not feel weakness of limbs, now blood glucose dropped to 5.3 mmol/L. The patient also suffered from hypertension, SBP 160-170mmHg, DBP 100-110 mmHg. After the patient wore the shoes for a month and more, SBP was 120-130 mmHg, DBP 70-80-90 mmHg. His hobby is fishing; previously his lower limbs were cool. After wearing the shoes, if standing on the ice, others would stomp for warming up, the patient need not stomp, and legs are now warm and quite nice.

### Example 21:

Wang Yufen, 60 years old, began to wear the shoes in 2008 who lives in Guofanglou community, Guofang road, Tangshan city. She had suffered from diabetes. Blood glucose was 7.8mmol/L. After the patient wore the shoes for a month, now blood glucose is always controlled to 5.4 mmol/L. Originally Wang's heart rate was so fast that maximum heart rate was 120 beats/min. After Wang wore the shoes for two months, normally Wang's heart rate is always 75-80 beats/min. Wang also suffered from hypertension, SBP 180 mmHg, DBP 100 mmHg. After the patient wore the shoes for three months, SBP and DBP were always controlled to 140/80 mmHg. Wang also had suffered from cervical spondylosis, could not open eyes, and could not lift up his arms at all. After wearing the shoes for a month and a half, Wang could open her eyes, move the arm, turn the neck easily and there was not a sound anymore when she turned her neck.

### Example 22:

Lifeng Zhi, 67 years old, began to wear the shoes in 2011 who lives in Guye district, Tangshan city, Zhaogezhuang village. She had suffered from diabetes. Blood glucose 20.0 mmol/L, U-GLU ++++. After the patient wore the shoes for 2 months, her blood glucose dropped to 5.8 mmol/L, U-GLU+ disappeared. Li suffered from hypertension, SBP 220mmHg, DBP 110 mmHg. After the patient wore the shoes for 2 months, SBP dropped to 130mm Hg, DBP to 85mmHg.

### Example 23:

Jia Wanshun, 74 years old, began to wear the shoes in 2011 who lives in Ronghuabeilou, Henanli, Tangshan City. He had suffered from diabetes. Blood glucose was 10.05 mmol/L. After the patient wore the shoes for two months, his blood glucose dropped to 5.7 mmol/L. Previously when his one hand touched the other hand, he would have the feeling of tingling, he also had blurred vision. After the patient wore the shoes for two months, these symptoms disappeared. Jia also suffered from coronary heart disease, chest distress, shortness of breath, premature beats and angina. After the patient wore the shoes for two months, these symptoms disappeared. The patient suffered from bradycardia 54-55 beats/min. After the patient wore the shoes for two months, heartbeat increased to 65 beats/min or more. Jia also suffered from arthritis, especially in the knees, he had to grab the railing when he went upstairs. After the patient wore the shoes for two months, it is convenient for him because it is not necessary to grab railing to go upstairs, even he could carry things as heavy as 4 kilos or 5 kilos.

### Example 24:

Meng Xianrong, 62 years old, began to wear the shoes in 2010 who worked in Research Institute of Machinery Bureau, Tangshan city. He had suffered from diabetes. Blood glucose was 10.0 mmol/L. He felt itching in the whole body, and had a blurred vision. After the patient wore the shoes for one month and a half, blood glucose dropped to 6.5-6.6 mmol/L, vision of the eyes turned better, itching in the whole body was also improved. Meng had suffered from periarthritis of shoulders, she could not lift up her right arm, and it was difficult to comb her hair. After wearing the shoes for two months, she could lift her arms wherever she wanted to lift. Meng also had suffered from painful legs, when she wanted to squat, she had to slowly squat bit by bit grinding her teeth, once squatted, she could not stand up. After wearing the shoes for two months, she could squat and stand up easily.

### Example 25:

Chen Zhijun, 59 years old, began to wear the shoes in 2006 who lives in 34. Sanyuantang, Changshu city. He had suffered from diabetes. Blood glucose was 24.8mmol/L, U-GLU ++++. After the patient wore the shoes for two months, blood glucose was controlled at about 5.0 mmol/L, and U-GLU+ disappeared. Chen also had suffered from coronary heart disease and angina. Presently these symptoms basically disappeared. After Chen fell sick with diabetes and heart disease, he had edema on his two feet and two hands every evening; he would take discutient once a week. Presently, he does not have edema on his two hands and two feet in the evenings.

### Example 26:

Zhang Baoxing, 60 years old, began to wear the shoes in 2007 who lives in fifth group, Xu Zhen village, Zhitang town, Changshu city. He had suffered from diabetes, hypertension and periarthritis of shoulders. Blood glucose was 13mmol/L and more, After the patient wore the shoes, his blood glucose dropped to over 6.0 mmol/L. Previously the SBP was 160mmHg, DBP 120mmHg. After the patient wore the shoes, SBP dropped to 120 mmHg, DBP to 80 mmHg. Previously as Zhang worked, it was very painstaking and very painful when he turned around. After the patient wore the shoes, gradually the shoulders became agile and flexible.

### Example 27:

Yao Zhidong, 62 years old, began to wear the shoes in 2006 who lives in Jinhe Jiayuan 1 district, Changshu city. He had suffered from diabetes, hypertension and periarthritis of shoulders for five years. Blood glucose was 12.7mmol/L. After the patient wore the shoes, his blood glucose dropped to about 7.0 mmol/L. The original SBP and DBP were 160/100mmHg, after the patient wore the shoes, they dropped to 140/80 mmHg, and periarthritis of shoulders has been relieved remarkedly.

Example 28: Ji Yongming, 45 years old, began to wear the shoes since 2006 who lives in Qinhu fourth districts 32-3, Changshu city. He had suffered from diabetes. Blood glucose was 8.9mmol/L. After the patient wore the shoes for a month, his blood glucose dropped to about 6.5 mmol/L. After the patient wore the shoes for two months, his blood glucose dropped to 4.0 mmol/L. Ji has been wearing the shoes until now, his blood glucose was stabilized at about 4.0 mmol/L.

### Example 29:

Wang Zailiang, 63 years old, began to wear the shoes in 2007 who lives in Xiyan village, Changshi town, Changshu city. He had suffered from diabetes for 15 years. Blood glucose was 11.7mmol/L, postprandial blood glucose was more than 20 mmol/L, U-GLU ++. After the patient wore the shoes for two months, fasting blood glucose dropped to 4.7 mmol/L, postprandial blood glucose 8.5 mmol/L, U-GLU +. Wang also had suffered from hyperension, SBP 170mmHg, DBP 90 mmHg. After the patient wore the shoes for three months, SBP dropped to 140 mmHg, DBP dropped to 75 mmHg.

Example 30: Guo Yuda, 80 years old, chief physician, professor, began to wear the shoes since 2007 who lives in Third Military Medical University, Changchun City, Jilin Province. He had suffered from hypertension, SBP 200mmHg. After the patient wore the shoes for more than one month, presently the Blood pressure is 120/80mmHg. Guo also had suffered from cervical spondylosis, the motion of the neck was limited, in the past Guo could not lower his head, if he did so he would felt sick and wanted to vomit. Since the patient wore the shoes these symptoms has been relieved. The patient used to urinate 6-7 times a night; sometimes he would urinate one time per hour, so he could not sleep well. After wearing the shoes, he urinates 1-2 times a night now.

### 2. The body self-healing and recuperating shoes, like penicillin, have the special effects of treating several diseases simultaneously with high recovery rates:

In 1928, Alexander Fleming discovered penicillin. In 1945, University of London Professor of Bacteriology, Sir Alexander Fleming; Oxford University's Biochemist Ernst Boris Chain and Professor of Pathology Sir Howard Walter Florey, three scientists shared Nobel Prize in physiology or medicine.

Since the advent of penicillin in 1945, it made a significant contribution to mankind for the treatment of infectious diseases. In the past the diseases influenza pneumonia, pleurisy, meningitis, nephritis, acute appendicitis, complications of surgery or trauma infection and others, resulted in the death of hundreds of millions of people. Regardless of severity and age, as long as the patients might use penicillin, they would be cured without exceptions (apart from allergies).

Like penicillin eliminated the common etiology of acute diseases: bacterial infection; the body self-healing and recuperating shoes having medicinal magnetic therapeutic function eliminate the common etiology of the diseases in elderly: hypoxia. Therefore body self-healing and recuperating shoes also have the special effects of treating several diseases simultaneously with high recovery rates.

The body self-healing and recuperating shoes having a medicinal magnetic therapeutic function can repair and enhance the body self-healing capability, thus lead to the self-healing of hypoxic disease in elderly. The body self-healing and recuperating shoes are global origination, which is a world-famous new idea of medical treatment. This is a new medical approach to treat several diseases simultaneously, which offers a new thinking and opens up a new way for the ideal of human health and longevity. The shoes realize humanity's dream of "treating diseases without taking medicine". The body self-healing and recuperating shoes will make it easily to realize a topic about human health and longevity. The body self-healing and recuperating shoes can make every patient get rid of the trouble of the disease and get well soon. The shoes have made a significant contribution to human health.

The application of the body self-healing and recuperating shoes are simple and practical, save time, and play an unusual role in the ordinary life. The shoes are affectionately called as the health envoy, the personal doctor by the elderly patients.

### 3. the self-healing range of the body self-healing and recuperating shoes and the course of treatment:

Self-healing in three months:
Under the self-healing function of body self-healing shoes, the patients are able to stop using other therapeutic drugs on the same day.

In the 10th day, one third of various kinds of inflammatory swelling and pain in the elderly can be improved. In a month, half of their swelling and pain can be improved. In three months, various types of inflammatory swelling and pain such as cervical and lumbar spondylosis, periarthritis of shoulders, rheumatic and rheumatoid arthritis (in six months, self-healed) can be eliminated, the function of secretion of joints fluid by joint synovial may return to normal. Various symptoms of hypoxic lesions such as hypoxic hyperostosis of the cartilage cells in cervical vertebra, lumbar vertebra, shoulders, hands, legs and feet and knees, and their deformation, lumbar intervertebral disc rupture, nucleus pulposus prominent, joint stiff, swell and soreness, asthma, insomnia, constipation, prostatic hyperplasia, neurodermatitis, intense itching skin, as well as functional disorders such as insomnia, limited activity, difficulty in walking and going up and down the stairs and so on can be regeneratively repaired and self-healed.

Skeletal deformities can be improved very markedly, but the bones with organic lesions cannot be restored.

Before wearing the shoes, the patients can first go to a hospital to have a blood routine test on counts of red cells, white cells and platelets, so that a successive contrast comparison can be made.

Treating with the body self-healing and recuperating shoes, every one month the patient should undergo a blood test. In three months, blood viscosity, blood lipids, blood glucose, blood pressure, contents of creatinine, lactic acid, uric acid, urea nitrogen will return to normal; individual numbers as well as maturation rates of red cells, white cells, platelets, and physical and chemical indices will turn to normal.

In six months those diseases listed below can be self-cured:
In 10 days, chest distress, shortness of breath, dizziness and headache can be improved preliminarily.

In a month, chest distress, shortness of breath, dizziness, and headache can be improved markedly.

In three months, chest distress, shortness of breath, dizziness and headache can be completely eliminated.

In six months, hypoxic lesions such as diabetic cardiovascular diseases, diabetic hypoxic bone marrow membrane impatency, and anoxic hematopoietic function disorder of bone marrow stem cells (except those patients with complete stem cells fibrosis), diabetic nephropathy syndrome, glaucoma, peripheral nerve inflammation, other complications such as skin itching, and secondary nephrotic syndrome (including renal transplant patients), coronary heart disease, bradycardia, hypotension, anemia, hypertension, etc. can be regeneratively repaired and self-healed.

In six month those diseases listed below can be self-cured or improved very markedly:
In 6 months, patients with hemiplegia, walking off course, motion difficulties, failure to take care of themselves, incontinence and other sequelae of cerebral infarction as well as stroke can also be self-cured or improved very markedly. Patients with crutches, in wheelchairs, and the paralyzed will be able to walk short distances independently. Even those suffer from more than 10 kinds of diseases, eight kinds can be self-healed (after 3 courses), and two kinds can be improved markedly (after 6 courses).

Less than 3% diseases failed to be self-healed:
① owing to too many kinds of illness suffered by the patients, suffering from diabetes and cardio- and cerebro-vascular disease simultaneously.
② owing to the severity of the illnesses suffered by the patients, two kinds of the diseases has reached phase III.

### ② owing to the relatively old age of the patient, they have been more than 70 years old.

However, no matter what kind of problems the patients have, as long as they are not yet hospitalized to be salvaged, if they continue to use body self-healing and recuperating shoes for more than 3-6 months on the premise that they can normally use the shoes, even the patients suffer from as many as 10 kinds of diseases, 8 kinds can still be self-healed, two kinds can be improved very markedly.

### Important relationship of health with feet

Senility begins with the age; aging starts from the kidney. Aging of kidneys starts from the blood; aging of blood start from the feet. To say aging of kidneys starts from blood, it is because kidney is the only organ which filters lactic acid, uric acid, urea nitrogen, creatinine and other metabolic garbage out of blood into urine. Therefore, kidneys are damaged by these toxic substances all the time. Therefore the earliest failure of organs in the human body that appears is kidney failure. Thus, the aging of kidney starts from blood.

To say aging of blood starts from the feet, it is because that every day feet bear the heaviest burden of human body and the pressure of activities, With the increase of age, more metabolic garbage and toxins accumulate in the feet, these toxins will be circulated into the blood in the feet, resulting in long-term damage of the blood, and leading to aging of blood. Therefore, the aging of blood starts from the feet.

It is because of body self-healing and recuperating shoes found the feet as the point of breakthrough to remedy diseases, the aging blood problem has been solved fundamentally by recuperating treatment of feel in human body.

Under the effect of the body self-healing and recuperating shoes, electromagnetic energy in red cells is more sufficient, the spiral walk is faster, the clogged kidney capillaries can be dredged by red cell, the blood circulation in kidney capillary balls will be unobstructed and exuberant, the function of kidneys to filter urinary can be restored, lactic acid, uric acid, urea nitrogen, creatinine and other metabolic wastes and toxins accumulated in the feet can follow unobstructed and exuberant blood circulation to reflux from the feet, passing through the kidneys, finally to be filtered out. Thus the problem of aging of human blood has been fundamentally resolved. Above information about kidney diseases as well as important relationship between feet and health are from clinical medicine all around the world.

### Clinical Medicine: Hypoxic bone marrow membrane impatency and blood routine examination:

http://baike.baidu.com published an article on medical science in 2011, stating that acute hypoxia is life-threatening, chronic hypoxia could threaten health. But the vast majority of people do not know those.

Human blood has more than 20 trillion red cells; their average lifespan is 100-120 days. When number of red cells decreases, the supply of oxygen is reduced, and then the body in a hypoxic state, leading to anemia; when it gets severe it will be life-threatening. Whereas the number of red cells in the elderly is a lot fewer than in that of the young people, and the lifespan of the blood cells a lot shorter, so the oxygen in the bodies of elderly is insufficient. With their increasing age, the oxygen content in the bodies of elderly will be more inadequate.

Thus, hypoxic marrow membrane impatency caused by chronic hypoxia is the crucial lesion that first appears in every elderly. When blood perfusion in human skeleton endometrial capillaries is blocked by more than 65 percent, then it is known as hypoxic bone marrow membrane impatency.

When bone marrow film hypoxic impotency happens, hypoxic hematopoietic stem cells will lead to hypoxic hematopoietic functional disorder; numbers of red cells, white cells platelets produced by bone marrow stem cells will be reduced. The mature proportions of the red cells, white cells, and platelets in elderly are already low, with hypoxic hematopoietic functional disorder, in vivo hypoxia problem in the elderly will be even severer, that will result in low immunity, and frequent colds.

Therefore, the blood routine test for the elderly in the hospitals is a very important project.

Because only after the routine tests on blood viscosity, red cells, white cells and platelets, can the correct diagnosis be made by the doctors according to the analysis on blood anomalies, and subsequently, correct treatment of illness in the elderly.

But no matter how the geriatric diseases are treated, they can only be protracted, causing recurrence of the disease after treatment, treatment after recurrence of the disease. The reason is that the core lesion of the onset of geriatric diseases, hypoxic bone marrow membrane impotency, has not been eliminated. Therefore the geriatric diseases cannot be healed.

Whereas the onset of hypoxic bone marrow membrane impatency is owing to natural decline of all physiological functions in the elderly.

When the elderly ages, capillaries in the whole body will gradually age and impatency will appear, the body's blood perfusion will be blocked. In particular, the number of capillaries in bones and bone marrow membranes is already 50 times less than those in other organs, therefore it is more likely to result in reduced blood and oxygen supply to bone marrow membranes, aggravate the problem of hypoxic bone marrow membrane impatency.

If the problem of hypoxic bone marrow membrane impatency is truly eliminated, the diseases in the whole body of the elderly will be self-healed naturally, and the ideal that elderly dies without a sickness can be achieved.

Blood viscosity can be measured by means of the hemorheological instrument in hospitals. The higher the read of red cells per unit volume, the higher the blood viscosity. Red cell ratio refers to the share of red cells in the blood volume. Normal red cell ratio was 45% for male, 40% for female.

Blood routine tests of counts of white cells, platelets are equally important. The total amount of white cells in the human body is more than 50 billion, with an average life expectancy of 1-12 days. The reason why the elderly frequently catch cold is because of the fewer leukocytes leading to reduced immunity. On the other hand, when the number of white cells increases, it is because non-specific inflammation appears in the bodies of the elderly; that is reason of occurrence of various types of inflammative swelling, sore and recurrent low fever.

The platelets are vascular maintainer of human whole body; there are more than 2 trillion platelets in a human's blood.

When the amount of blood platelet is no less than 300,000 per deciliter of blood in the human body, the integrity of the whole vascular intima can be maintained. When the amount of blood plateletis less than 20,000 per deciliter of blood, the problems, such as gastrointestinal bleeding, cerebral hemorrhage, hematuria and so on, will happen, and they will threaten the life of the patient. Using the transmission electron microscope, the ultrastructure or unusual status of white cells, platelets and various types of cells in the human body, with only a few microns of their sizes which are unable to see with the naked eyes, can be magnified to the sizes of eggs, thus they can be observed very clearly. For ultrastructure patterns of human cell types, please refer to clinical books.

When human magnetic field enhances, human bio-electricity in erythrocytes can also be enhanced.

Medical research in the world shows that the human bio-electricity in the red cells under the strong magnetic field is enhanced in a strong magnetic field model. Such experiments show that the more human bio-electricity the red cells carry, the more abundant is the electromagnetic energy in the red cells, the higher is the electrophoretic rate of the red cells, the faster are their spiral walk. With their diameter of 8 microns, the red cells can clear capillaries of the diameters of 6-8 micron smoothly one by one, the aged, blocked capillaries in the body can be dredged by red cells, following the red cells, blood perfusion can be fluent in more than 400 million capillaries of the whole body, the blood circulation in whole human body as well as in the bones and bone marrow membranes will be unobstructed and exuberant, when the blood circulation in the bones and in bone marrow membranes is unobstructed and exuberant, bones and bone marrow membranes will not suffer from hypoxia, there will be no hypoxic bone marrow membrane impatency

Bones are the body's framework, bone marrow stem cells are the hematopoietic cells for the production of red cells, white cells and platelets.

Under the effect of the body self-healing and recuperating shoes hypoxic bone marrow membrane impatency can be eliminated, except for the cases in which bone marrow stem cells are entirely fibrotic.

Under effect of the body self-healing and recuperating shoes, the more sufficient is human bio-electricity on the surfaces of the red cells, the higher is the negative charge density on red cell membrane surface, and the more is the number of oxygen molecules red cells can carry. Then the red cells can attract sufficient divalent iron ions, to be adsorbed and gathered in the cell membrane under the effect of electromagnetic hydrodynamic attraction, then red cell can produce enough oxygen-carrying hemoglobin molecules needed. More than 1 billion hemoglobin molecules in each red cell can combine with more than 4 billion oxygen molecules, to form oxygenated hemoglobin in lung alveoli, each red cell can carry more than 4 billion oxygen molecules, more than 20 trillion red cells can carry and transport more than 1×10²² oxygen molecules per hour, more than 500 trillion cells in human body can get sufficient supply of more than 1×10²² supplementary oxygen molecules per hour, the whole body's oxygen supply can be guaranteed, the blood oxygen levels in human body will rapidly increase, hypoxic bone marrow membrane impatency and hypoxia functional disorder of bone marrow hematopoietic stem cells will be eliminated. When sufficient oxygen is in the body, bone marrow stem cells are excited and active, hematopoietic function of the bone marrow stem cells to produce red cells, white cells and platelets is improved, the deficiency of blood cells can be adequately supplemented anywhere and at any time. Thus the physiological function of the red cells, white cells and platelets in the bodies of the elderlies can always be maintained into a healthy and strong status.

Before adopting body self-healing and recuperating shoes, the patients are suggested to go to a hospital first to do a blood routine test on counts of red cells, white cells and platelets, so that a contrast successive comparison can be made. Under the effect of the body self-healing and recuperating method, once a month a routine blood test can be implemented. In three months hospital test would show that individual counts of red cells, white cells and platelets as well as their mature rates can be restored.

The grounds of reasoning relating to hypoxic bone marrow membrane impatency and blood routine examination are from clinical medicine all around the world.

### Clinical medicine: the principle of self-healing capability of the human being and the recuperating and self-healing by the shoes:

WHO appeals to get rid of the dependence on the drug, thus, in order to possess a real health status, we should enhance the body's own healing capability, repair various human organs' functions, and maintain and recovery the body's own capability of independent health. This is the call of the destiny of mankind, also will become the future development trend of medicine.

Experts in the preventive medicine profession believe that in the treatment of the disease using various drugs, their side effects are damaging patients' body function and accelerate the aging of patients, as a cost of seeking temporary equilibrium in the lesion site. Even highly advanced modern medicine does not cure the disease in the true sense; as a result, often side effects of drugs accelerate aging of human cells and tissues. In fact, oral administration of drugs should play their role by adjusting the self-heal system of the body. If we say that the side effects of the drug only interferes with the normal regulation and balance in the body, then the long-term use of drugs which would generate drug resistance will result in even more diseases that the patient is suffering presently, and even less effective the drugs will be. The highest level of medicine is to repair and enhance the self-healing capability of the body.

http://baike.baidu.com published an article on medical science in 2011, stating that healing capability are like the root of a tree, when the root is strong, withered and yellow leaves on whole crown of the tree will turn to lush foliage. When the body's self-healing capability is enhanced, diseases in whole body will be cured. When the body healing capability reduced, diseases and aging will arise. Therefore, enhancing and improving the healing capability are the key to disease rehabilitation. In the process of the rehabilitation of patients, less practical role is played by doctors and medicines, it relies more on self-regulation, which is a process of repairing self-healing capability. After the self-healing capability is improved, not only accelerate rehabilitation from the disease, but also save a lot of medical expenses. The drug therapy will spend money endlessly, whereas repair of human self-healing capability spends only limited money.

Human body self-healing capability refers to the capability of the body's natural healing, which is the capability of self-regulating everyone has.

Depending on the body self-healing capability, human life is maintained in a healthy status, the damage of organs and tissues can be repaired, and life can be continued. The connection of bone fractures, healing of soft tissue trauma, mucosal cell regeneration and replacement in glands, endocrine regulation, killing of bacteria and viruses by immune system, these are some examples of the self-healing capability of inherent spontaneous action of regenerative repair, the capability to self-heal. The repair and upgrade of self-heal capability of the body, however, need plenty of oxygen.

http://baike.baidu.com published an article on medical science in 2011 stating that only when human tissue cells obtain enough oxygen supply can they work properly. The metabolism of tissue cells needs plenty of oxygen. Human tissue hypoxia will cause all the biochemical reaction processes into incomplete reactions, as a result producing corresponding toxins. This question is actually the root cause of all chronic diseases. Appropriately supplementing oxygen, then it is possible to resulting in the recovery of diseases through the repair and upgrade of the self-healing capability of the body.

Oxygen, when erythrocytes pass through the blood vessels of the foot once in every more than 20 seconds to form vertical circulation during the circulation of the blood, will cut magnetic lines of force in the Earth's magnetic field, result in the electromagnetic hydromechanics phenomenon, and micro electric current is produced. That is, only when the human body has bio-electricity, can the red blood cells carry oxygen.

It is difficult to supply the human bio-electricity from other parts of the body.

Only in the process during human walks with the shoes will he be able to naturally obtain supplement of human body bio-electricity.

Feet are the only part of the body which can obtain the supplement of body bioelectricity in contact with the Earth's magnetic field. There is a common saying known as "being contacting with ground *Qi",* it refers to the feet contacting with the Earth's magnetic field.

The longer one flies in a plane, the more one's legs and feet feel swelled and have the feeling of tired and hypodynamia. This is because the farther away the plane is from Earth's magnetic field, the more insufficient the body obtains ground *Qi,* causing the red cells' failure to be supplemented with human bioelectricity. With a diameter of 8 microns, the red cells should have difficulties to dredge the capillaries with diameters of 6-8 micron, and furthermore, lead to blocked blood perfusion in human whole body capillaries, causing swelling of legs and feeling of tired and hypodynamia.

Self-healing body self-healing and recuperating shoes can simulate the Earth's magnetic field, to supplement human body with human bio-electricity.

The body self-healing and recuperating shoes having medicinal magnetic therapeutic function aim at the part of feet, which is the part in human body farthest from viscera and is the safest, as object of supplementing human bio-electricity, using shoes as physiotherapy carrier, which are provided with optional number and size of the containers in the shoes, as a result a special set of energy fields are formed inside the shoes. When the red cells containing ferroprotein circulate with blood flow once in every 20 seconds passing through the energy field, they cut the magnetic lines of force and thereby resulting in electromagnetic hydrodynamic phenomena and generating microcurrent, i.e. human bio-electricity. The bio-electricity of 20 trillion human red cells can get 180 times of adequate complement per hour, the degree of excitement and activity of the red cells are enhanced, and their spiral walk will be faster, with their diameter of 8 microns, the red cells can dredge capillaries of the diameters of 6-8 micron. The aged, blocked capillaries are dredged by the red cells, following the red cells, blood perfusion can be fluent in more than 400 million capillaries of the whole body, the human body's blood circulation will be unobstructed and exuberant, the problem of blocked blood flow and microcirculation is eliminated, and thus the whole body's blood supply can be guaranteed.

Under the effect of body self-healing and recuperating shoes having medicinal magnetic therapeutic function, human bio-electricity is abundant in red cells, under the effect of electromagnetic hydrodynamics attraction, red cells will be able to absorb and gather sufficient divalent iron ions into the cell membranes of red cells, and they can manufacture enough hemoglobin molecules needed for carrying oxygen. More than 1 billion hemoglobin molecules in each red cell can combine with more than 4 billion oxygen molecules, to form oxygenated hemoglobin in lung alveoli, each red cell can carry more than 4 billion oxygen molecules, more than 20 trillion red cells per hour can carry and transport more than 1×10²² oxygen molecules, more than 500 trillion cells in human body can get sufficient supply of more than 1×10²² supplementary oxygen molecules per hour, the whole body's oxygen supply can be guaranteed, the source of many diseases, lack of oxygen, can be eliminated. Oxidation, synthesis and conversion of blood glucose, fat, proteins, and cell metabolism will obtain enough energy and heat; the body's self-healing capability will be fully repaired and upgraded.

With sufficient oxygen, even if the defect or necrosis occur in human cells, the surrounding, same type of cells can proliferate by splitting, differentiation and regeneration, with timely regeneration and replacement of the new cells, the original structure and function of organs and tissues can still be completely restored, hypoxic lesions and functional disorders of organs and tissue in human body can get regenerative restoration, being self-healed.

Under the effect of body self-healing and recuperating shoes having medicinal magnetic therapeutic function, in 30 minutes in vivo blood glucose aerobic oxidation will take place with sufficient oxygen in the body of the patient, transform into ATP, and release heat, the patient's feet first feel hot, every day the body is like in a status of exercising a 10 km walk, the patient's metabolism is accelerated, he does not fear cold. The patient's constitution can be strengthened, immunity can be improved, is not easy to catch cold. The self-heal recuperating can also prolong life and delay aging.

The grounds of reasoning relating to human seal-healing capability of human body are from clinical medicine all around the world.

### Clinical medicine: human bio-electricity, oxygen and blood circulation:

The human body is consisted of more than 500 trillion various types of cells, the cells are constituted of proteins.

Each cell carries human body bio-electricity. Human bio-electricity is an important element with which the skin, muscles, nerves, bone marrow stem cells and other body cell types change their status from a standstill when they are at the resting potential, to an operating status when they are at the action potential, to exchange oxygen and nutrients through their membranes.

When a certain part of the body is stimulated, the body sensory organs will be excited; they will generate action potentials conducting along the afferent nerves. The excitements conduct to the brain, the brain will react according to the information passed by the excitement, issues a directive. Then the brain's instructions are conducted by the efferent nerves to the relevant effector organs, to complete the appropriate actions. During the process of conducting information, the excitement is the body bioelectricity.

Bio-electricity was first discovered by the Italian biologist Galvani in 1786.

This novel discovery of bio-electricity caused a great shock in the scientific field. US President Gerald Ford once commended that the discovery of bio-electricity by Galvani was one of the great epoch-making discoveries.

Human bio-electricity is the impetus to cells, with sufficient bio-electricity every cell of the human body will get exited, being impulsed and active. Heartbeat needs bioelectricity of 1-2 millivolt, thinking of brain needs 0.2-1 mV, opening and closing of eyes 5-6 millivolt to generate action potentials and excitement activities.

When abnormal change of potentials in human bioelectricity images such as ECG, EEG, MRI are observed, these can be used to diagnose the disease status and the extent of cardiovascular and cerebrovascular disease, as well as the related diseases.

Red cells are the transport brigade to dredge blood vessels, and transport blood and oxygen.

The majority of components of blood are red cells; there are more than 20 trillion of them. Reducing the number of red cells, the supply of oxygen is reduced, the body will be in a hypoxic state, anemia will come into being; When this state is severe, this will threaten life.

Each red cell carries with more than one billion hemoglobin molecule containing ferroprotein. Each hemoglobin molecule is composed of four heme molecules, in the center of each heme is a divalent iron ion, each hemoglobin molecule can be combined with four oxygen molecules.

The human bio-electricity plays a key role in maximizing the oxygen molecules being carried in the red cells, whereas the number of red cells to carry oxygen molecules is directly proportional to the number of hemoglobin molecules the red cells produce.

The more abundant human bio-electricity is in red cells, the higher is the density of their surface negative charge, the more the number of oxygen-carrying molecules they carry. Only then can the erythrocytes attract and gather sufficient divalent iron ions into their membranes under the effect of electromagnetic-hydrodynamics attraction, before they can produce enough oxygen-carrying hemoglobin molecule needed. More than 1 billion hemoglobin molecules in each red cell can combine with more than 4 billion oxygen molecules, to form oxygenated hemoglobin in lung alveoli, each red cell can carry more than 4 billion oxygen molecules, more than 20 trillion red cells can carry and transport more than 1×10²² oxygen molecules per hour, more than 500 trillion cells in human body can share sufficient supply of more than that number of supplementary oxygen molecules per hour, then the whole body's oxygen supply can be guaranteed.

These phenomena about human bio-electricity and density of red cell surface negative charge can be tested with cathode ray oscilloscope. Electrophoretic mobility of red cells can be test with capillary electrophoresis apparatus.

Erythrocyte electrophoretic rates refer to the migration velocities and time of red cells in blood.

For relating basic experimental arrangement and models, please consult clinical books.

Oxygen is the energy of cells. With adequate oxygen, every cell will have the strength to work.

With insufficient oxygen, oxidation, synthesis and conversion of blood glucose, fat and proteins will go wrong, excitement of nerve, movements of limbs, secretion by glandular, metabolism in cells will not obtain enough energy and heat to be carried on, the body self-heal capability will reduce, hypoxic lesions and functional disorders of human organs and tissues cannot be regenerated, hypoxic lesions or canceration will take place.

With sufficient oxygen, oxidation, synthesis and conversion of blood glucose, fat, proteins and excitement of nerves, movements of limbs, secretion by glandular, metabolism in cells will obtain enough energy and heat to be carried on, the body self-heal capability will be comprehensively repaired and upgraded.

With sufficient oxygen, even if pathologic defects or necrosis developed in the human organs and cells in the tissues, self-healing capability of the surrounding cells of the same type can still play a role. The cells will be supplied with enough energy and heat to proliferate through fission, the rate of regeneration and replacement will be accelerated. These timely regenerative replacements with the new-born cells will still be able to completely restore the original structures and functions of organs and tissues, hypoxic lesions and functional disorders of human organs and tissues can get regenerated and repaired, and self-healed, hypoxic lesions or canceration will not occur. It can be said that with insufficient oxygen, diseases will attack; with sufficient oxygen, diseases will fade away.

The most fundamental function of blood circulation is that through ceaseless exchange of oxygen and nutrients with every other human cell. This function is completed in the capillaries.

Capillaries are the only channels to exchange oxygen and nutrients between the blood and more than 500 trillion various types of cells in the body, but also the only places body completes the metabolism.

The blood circulation in the capillaries cannot rely only on heart beating to complete.

Blood circulation in the capillaries relies entirely on red cells with a diameter of 8 microns, in its state of sufficient human bio-electricity, to be curly deformed with spiral walk they dredge the capillaries of diameters of 6-8 micron. Each capillary can only accommodate single red cells to pass one by one.

During dredging of the capillaries by red cells, all the body's metabolic garbage generated by the metabolism of cells can reflux through blood circulation from the capillaries back to the veins along with red cells and blood, when they flow through the kidneys the metabolic garbage will be filtered out.

Human body is connected by more than 40 billion small arteries and capillaries; the total length is over 100,000 kilometers, with a total area of about 6,000 square meters. Inside the walls of arteries and veins with diameters of 1 mm or more are distributed with capillaries to supply nutrition to blood vessel wall. Inner diameter of capillaries is between 6-8 microns, the length is 0.2-4 mm. The capillary wall is very thin, it is only a layer of epithelial cells, the thickness of a capillary is one hundredth of the thickness of a sheet of paper, the diameters of 20 capillaries put together is just as thick as a hair.

With the increase of age of the elderly, the more clogged sites are in the capillaries.

Different diseases vary with the different sites of aging capillary blockage.

The severer the problem of capillary aging and being blocked, the severer the oxygen deficiency problem is.

The severer is the problem of oxygen deficiency, the heavier are the attacking diseases.

When the human bio-electricity is sufficient in the red cells, capillary blockage problem does not take place.

The more sufficient human bio-electricity is in the red cells, the higher is the degree of excitement and activity of the red cells, the higher the speed of their spiral walk. With their diameter of 8 microns, the red cells can drudge capillaries of the diameters of 6-8 micron smoothly, blood perfusion can be fluent in more than 400 million capillaries of the whole body, the human body's supply of blood can be guaranteed.

When the human bio-electricity in the red cells is insufficient, they cannot walk spirally to dredge the capillaries with diameters 2 microns finer than their own, capillary congestion problem occurs.

The grounds of reasoning relating to human bio-electricity, oxygen, blood circulation are from clinical medicine all around the world.

### Clinical medicine: the principle that diabetes can be recuperated by repairing and enhancement of the body self-healing capability:

Hypoxic glucose metabolism disorder is a major cause of diabetes.

With hypoxia, one mole of glucose will be able to generate energy of only two molecules, releasing only 1/19 of the energy and heat energy. Because under anaerobic conditions carbon-hydrogen bonds in glucose cannot be fully opened, aerobic oxidation of glucose will not take place, hypoxic sugar metabolism will take place in the body, leading to increased blood glucose and diabetes.

The experimental results published by the University of Manchester stated that due to chronic hypoxia and increasing anaerobic glycolysis, human tissue cells will produce increased amounts of lactic acid, which will eventually evolve into diabetes and secondary symptoms of hypoxia in more tissue cells, causing cardiovascular disease and various infections. Daily oxygenating will quickly enhance the blood oxygen concentration, and promptly correct hypoxia; this is an important supplementary means for the treatment of diabetes.

Aerobic oxidation is the main way of the body's glucose metabolism.

With sufficient oxygen, one mole of glucose can be oxidized with six oxygen molecules, generating energy equivalent to that of 38 ATP molecules, releasing 686kcal heat energy, and six molecules of carbon dioxide and six molecules of water. This is because in the oxygen-rich blood, glucose carbon-hydrogen bonds will completely open, generating aerobic oxidation. Blood glucose can generate sufficient energy and transform into heat, it will be metabolically consumed.

http://baike.baidu.com and bbs.tnbz.com published an article on medical science in 2012, stating that the latest medical research showed that the root cause of diabetes is chronic hypoxic of cells, chronic hypoxia of islet cells reduces the secretion of pancreatic insulin, and ultimately evolves into diabetes. Obviously, we should improve the oxygen supply to islet cells.

The body self-healing and recuperating shoes having medicinal magnetic therapeutic function can improve oxygen supply of islet cells, resulting in normal secretion of insulin. The body self-healing and recuperating shoes having medicinal magnetic therapeutic function aim at the feet as the objective to supplement human bio-electricity. The shoes are physiotherapy carrier, which are provided with optional number and size of the containers in the shoes, as a result a special set of energy fields are formed inside the shoes. When the red cells containing ferroprotein circulates with blood flow once in more than 20 seconds they pass through the energy field, they result in an electromagnetic-hydrodynamic phenomena of cutting the magnetic lines of force, generating micro-current, i.e. human bio-electricity. The bio-electricity of 20 trillion human red cells can get 180 times of adequate complement per hour, the degree of excitement and activity of the red cells are enhanced, speed of their spiral walk will be faster, with their diameter of 8 microns, and the red cells can dredge capillaries of the diameters of 6-8 micron, smoothly one by one. Following the red cells, blood perfusion can be fluent in more than 400 million capillaries of the whole body, the human body's blood circulation will be unobstructed and exuberant, the problem of blocked blood flow and microcirculation are eliminated, and blood supply in the whole body and pancreas can thus be guaranteed.

Under the effect of the body self-healing and recuperating shoes having medicinal magnetic therapeutical function, human bioelectricity in red cells is sufficient, the negative charge density in red cell surface is higher, under the effect of electromagnetic hydrodynamics attraction, red cells will be able to absorb and gather sufficient divalent iron ions into the cell membranes of red cells, they can manufacture enough hemoglobin molecules needed for carrying oxygen. More than 1 billion hemoglobin molecules in each red cell can combine with more than 4 billion oxygen molecules, to form oxygenated hemoglobin in lung alveoli, each red cell can carry more than 4 billion oxygen molecules, more than 20 trillion red cells per hour can carry and transport more than 1×10²² oxygen molecules, more than 500 trillion cells in human body can share sufficient supply of that number of supplementary oxygen molecules per hour, the whole body's oxygen supply can be guaranteed. The amount of insulin secreted by pancreatic islet β cells will turn to normal. When the body's blood oxygen levels increase rapidly, the hypoxic glucose metabolism can be eliminated. Blood glucose will undergo sufficiently aerobic oxidation, to be metabolically consumed. Blood glucose levels will turn to normal.

Under the effect of body self-healing and recuperating shoes having medicinal magnetic therapeutic function, oxygen is more sufficient in the body, the body self-heal capability will be comprehensive repaired and upgraded. With sufficient oxygen, even if the pathologic defects or necrosis take place in pancreatic islet β cells and endothelial cells of the kidney capillaries balls, the healing capability of the same kind of surrounding cells can still play a role. The cells can get enough energy and heat to proliferate through fission, the rate of regeneration and replacement will be accelerated. These timely regenerative replacements with the new cells will still be able to completely restore the original structures and functions of organs and tissues, hypoxic lesions such as diabetes, diabetic cardiovascular diseases, diabetes nephrotic syndrome, glaucoma, peripheral neuritis; severe itching of skin and functional disorders can get regeneration and repaired, being self-healed.

For people with no complications, the blood with sufficient oxygen, anaerobic glycolysis of blood glucose into lactic acid will not take place, diabetic complications will not occur.

With sufficient oxygen in the human body, lactic acid will react in vivo with weakly alkaline substance, sodium bicarbonate, converted into lactate, and water. In vivo blood glucose can react with abundant oxygen again with full aerobic oxidation, resulting in metabolic consumption. With unobstructed and exuberant blood circulation, sodium lactate and water will be filtered out as the blood carrying them flow through the kidneys, diabetic lactic acidosis will be eliminated.

Under the effect of body self-healing and recuperating shoes having medicinal magnetic therapeutic function:
After a month, the blood glucose can be decreased from about 20 mmol/L to about 10 mmol/L, U-GLU from (++++) to (++).

In two months, the blood glucose can be recovered to about 7 mmol/L from about 10 mmol/L, U-GLU (±).

In three months, blood glucose can be recovered within the normal range 3.9-6.1mmol/L, glycated hemoglobin can be recovered to 6.5%, U-GLU (-), blood lactate can be recovered to the normal level of less than 1.5mmol/L.

The grounds of reasoning relating to diabetes are from clinical medicine all around the world.

### Clinical medicine: the principle that coronary heart disease, hypertension, cerebral infarction, and stroke can be recuperated by repairing and enhancing the body self-healing capability:

Hypoxia is the common cause of coronary heart disease, hypertension, and cerebral infarction and stroke attacks.

http://baike.baidu.com published an article on medical science, stated that cardiovascular disease, diabetes and cancer have become top three causes of human death; their common feature is being caused by hypoxia in the body.

With insufficient oxygen, hypoxic damage and holes by focal loss of intima endothelial cells in arteries in heart and brain will happen, metabolism garbage and plasma lipoprotein in the blood will infiltrate through the damaged intima into the gap between the vascular membrane smooth muscle cells, adhered with elastic fibers and collagen, causing the removal reaction and infiltration of lymphocytes. To this end, vascular membrane smooth muscle cells in the blood vessel will generate an automatic defensive stress reaction to cause vascular hyperplasia, this generates cardiac and cerebral artery atherosclerotic plaques and plaques in whole human body as a result, leading to shrinkage of cavities in arteries in the whole body, and blocked blood flow, coronary heart disease, hypertension, cerebral infarction and cerebral hemorrhage.

When the human bio-electricity is insufficient, electrophoretic mobility of red cells will be reduced; the spiral walk will be slow. The electrophoretic mobility of the red cells of patients with coronary heart disease, myocardial infarction, cerebral infarction and stroke etc. are below normal, their spiral walk is slow.

When some of red cells are short of human bio-electricity, opposites attraction between the red cells whose membranes are charged with low- and high surface negative charge density, respectively, will gather and stack into bunches like bunches of grapes. Grape bunch-shaped red cells cannot pass the capillaries, these red cells and a lot of blood will remain in the arteries, the volume of blood occupied by red cells will exceed 45% of the normal value, causing increased blood viscosity, clogging arteries and small capillaries, and this will cause blocked blood flow and obstacle in microcirculation in more than 400 hundred million capillaries in the whole body, thus blood pressure will increase.

The body self-healing and recuperating shoes having medicinal magnetic therapeutic function can remove the obstacles to the blood flow, turning the blood pressure to normal.

The body self-healing and recuperating shoes having medicinal magnetic therapeutic function act on the feet as object of supplementing human bio-electricity, with the shoes as physiotherapy carrier, which are provided with optional number and size of the containers in the shoes, as a result a special set of energy fields are formed inside the shoes. When the red cells containing ferroprotein circulates with blood flow, once in more than 20 seconds passing through the energy field, they cut the magnetic lines of force and thereby resulting in electromagnetic-hydrodynamic phenomena and human bio-electricity. The bio-electricity of 20 trillion human red cells can get 180 times of adequate complement per hour, thereby increasing the red cell surface negative charge density, as a result red cells will repulse each other due to like charge, finally the problem that, due to the attraction between opposite charges, the cells aggregate into bunches like stacked chain to increased blood viscosity, can be eliminated.

When the body bioelectricity of the red cells is adequately supplemented, their electrophoretic mobility is enhanced, the speed of their spiral walk will be faster, with their diameter of 8 microns, the red cells can clear capillaries of the diameters of 6-8 micron smoothly one by one, the aged , blocked capillaries can be dredged by red cells, following the red cells, blood circulation perfusion can be fluent in more than 400 million capillaries of the whole body, the human body's blood circulation will be unobstructed and exuberant, the problem of blocked blood flow and microcirculation are eliminated, blood pressure is thus back to normal.

Under the effect of body self-healing and recuperating shoes having medicinal magnetic therapeutic function, the human bio-electricity on the surfaces of the red cells is sufficient, then sufficient numbers of divalent iron ions can be adsorbed and gathered in the cell membrane under the effect of electromagnetic-hydrodynamic attraction, red cell can produce enough oxygen-carrying hemoglobin molecules needed. More than 1 billion hemoglobin molecules in each red cell can combine with more than 4 billion oxygen molecules, to form oxygenated hemoglobin in lung alveoli, each red cell can carry more than 4 billion oxygen molecules, more than 20 trillion red cells can carry and transport more than 1×10²² oxygen molecules per hour, more than 500 trillion cells in human body can share sufficient supply of more than 1×10²² supplementary oxygen molecules per hour, the whole body's oxygen supply can be guaranteed. Hypoxia, the source of various diseases, can be eliminated.

With sufficient oxygen, intima endothelial cells in heart cerebral artery can get enough energy and heat for regeneration and repair, the body self-healing capability can be fully repaired and upgraded.

Under the effect of body self-healing and recuperating shoes having medicinal magnetic therapeutic function, oxygen supply to the human body will be guaranteed, new-born cells can be supplied with enough energy and heat, the proliferation by division, regeneration and replacement will accelerate, Hypoxic focal loss damage of cardiovascular artery intima can get regenerated by new-born endothelial cells, completeness of heart cerebral artery intima will be restored. Hypoxic cardiovascular diseases such as coronary heart disease, hypertension, cerebral infarction, stroke and functional disorders will get regeneratively repaired, and self-healed.

Under the effect of body self-healing and recuperating shoes having medicinal magnetic therapeutical function, red cells will carry oxygen and body bioelectrical to adequately supplement to more than 500 million white cells following unobstructed and exuberant blood circulation, white cells will be excited and active, their sizes increase, the function of alkaline phosphatase enzyme in cytoplasm of white cells to enzymatically hydrolyze atherosclerotic plaques will be improved. Heart cerebral artery atherosclerotic plaques, stroke plaques can be resolved by phagocytosis of white cells; problems such as narrowing of cardio-cerebral arterial lumen, blockage of blood flow, oppression of the brain cells by cerebral blood stasis can be eliminated, blood circulation in cardio-cerebral artery will be unobstructed and exuberant, supply of blood and oxygen can be guaranteed, the brain's commanding and regulatory function will return to normal, physical activity of the patients can be substantially free. Cerebral infarction and stroke sequelae such as hemiplegia, walking direction deviation, difficulty to move around, inability to take care of themselves, incontinence and others can be self-healed or improved very markedly. Patient with crutches, wheelchairs, or the paralyzed will be able to walk short distances independently.

The grounds of reasoning relating to coronary heart disease, hypertension, cerebral infarction, and stroke are from the clinical medicine all around the world.

### Clinical medicine: the principle that cervical and lumbar spondylosis, periarthritis of shoulders, rheumatic and rheumatoid arthritis can be recuperated by repairing and enhancing the body self-healing capability:

Lack of oxygen is the common pathogenesis of cervical and lumbar spondylosis, periarthritis of shoulders, rheumatic and rheumatoid arthritis. When the human bio-electricity in the red cells is insufficient, the electrophoretic mobility of red cells is low, the speed of their spiral walk will be low, the number of unimpeded capillaries will reduce, some red cells cannot enter the capillaries inside joints, the blood perfusion to the joints will be blocked, uric acid sodium salt and inflammatory pain-caused substances will accumulate in the joints, there will be large area of joint inflammatory pain and swelling.

When the blood flow in bones and joints is blocked, it will cause microcirculation disturbance and ischemia and hypoxia. In the cases of lack of oxygen, hypoxic bone hyperplasia and deformation will happen in cartilage cells in cervical and lumbar vertebra, shoulders and hands, legs and feet and knees, lumbar intervertebral disc rupture, vertebral pulp prominent and various hypoxic functional obstacle will happen as well.

When oxygen is insufficient, synovial function of synovial fluid secretion by joints is reduced, resulting in reduced secretion of synovial fluid, synovium injury, cervical and lumbar spondylosis, periarthritis of shoulders, rheumatic and rheumatoid arthritis, or hypoxic necrosis of caput femoris and other degenerative diseases.

Body self-healing and recuperating shoes can filter off pain-causing inflammatory material from joints, thereby eliminating joint inflammatory swelling and pain. The body self-healing and recuperating shoes having medicinal magnetic therapeutical function act on the feet as object of supplementing human bio-electricity, with the shoes as physiotherapy carrier, which are provided with optional numbers and sizes of the containers in the shoes, as a result a special set of energy fields are formed inside the shoes. When the red cells containing ferroprotein circulates with blood flow once in more than 20 seconds passing through the energy field, they cut the magnetic lines of force and thereby resulting in electromagnetic-hydrodynamic phenomena and human bio-electricity. The bio-electricity of 20 trillion human red cells can get 180 times of adequate complement per hour, the degree of excitement and activity of the red cells are enhanced, the speed of their spiral walk will be faster, with their diameter of 8 microns, the red cells can clear capillaries of the diameters of 6-8 micron smoothly one by one, the aged, blocked capillaries can be dredged by red cells, following the red cells, blood perfusion can be fluent in more than 400 million capillaries of the whole body, the human body's blood circulation will be unobstructed and exuberant, uric acid sodium salt and inflammatory pain causing substances accumulated in the joints will be refluxed following the unobstructed and exuberant blood circulation, filtered out through kidneys, as a result the inflammatory swelling and pain can be eliminated.

Red cells are the transport brigade in the body to drudge blood vessels, transport blood and oxygen.

Under the effect of the self-healing body self-healing and recuperating shoes, the human bio-electricity in the red cells will be sufficiently supplemented, the red cells will be able to absorb and gather sufficient divalent iron ions into their cell membranes, and they will be able to produce enough hemoglobin molecules needed to carry enough oxygen. More than 1 billion hemoglobin molecules in each red cell can combine with more than 4 billion oxygen molecules, to form oxygenated hemoglobin in lung alveoli, each red cell can carry more than 4 billion oxygen molecules, more than 20 trillion red cells can carry and transport more than 1×10²² oxygen molecules per hour, more than 500 trillion cells in human body can get sufficient supply that number of supplementary oxygen molecules per hour, the whole body's oxygen supply can be guaranteed, the source of many diseases, hypoxidosis, would be eliminated.

http://baike.baidu.com published an article on medical science in 2011, it stated that only when cells in human tissue get supplied by enough oxygen can they work properly, and that the metabolism of tissue cells need plenty of oxygen. Hypoxia will result inincompleteness of all the biochemical reaction process in human tissue, as a result toxins will be produced correspondingly. This is in fact the primary cause of all chronic diseases. By appropriate oxygenation, many diseases can be cured through restoration and upgrading of the healing powers of the body.

With sufficient supply of oxygen, even if pathologic defects or necrosis developed in cells in the joints, self-healing capability of same type of the surrounding cells can still play a role in regeneration and repair of joints. In that case various types of cells will obtain enough energy and heat to proliferate through fission, the rate of regeneration and replacement will be accelerated.

Under the effect of body self-healing and recuperating shoes having medicinal magnetic therapeutic function, self-healing capability of joints and the function of secretion of synovial fluid by joint synovium can be comprehensively repaired and upgraded. Hypoxic hyperostosis of the cartilage cells in cervical vertebra, lumbar vertebra, shoulders, hands, legs and feet and knees, deformation, lumbar intervertebral disc rupture, nucleus pulposus prominent will be subject to effects of proliferation by fission, regeneration and replacement of various kinds of joint cells. Hypoxic functional disorders such as cervical spondylosis, lumbar spondylosis, periarthritis of shoulders, the stiffness, swelling and soreness of joints caused by rheumatic and rheumatoid arthritis, as well as limited activity, leg cramps, difficulties in walking and going up and down the stairs and so on can be regeneratively repaired and self-healed.

Skeletal deformities can be improved very markedly, but the bones already have organic lesions cannot be restored.

The grounds of reasoning relating to the diseases in neck, shoulders, waist and legs are from clinical medicine all around the world.

### Clinical medicine: principle that kidney disease can be recuperated by repairing and enhancing the body self-healing capability:

Hypoxic is the major cause of kidney disease. Kidneys are composed of more than 2 million nephrons (capillary balls). When kidneys suffer from hypoxia, hypoxic spasm and impatency of kidney capillary balls will happen, lactic acid, uric acid, urea nitrogen, creatinine and other metabolic wastes and toxins will not be timely filtered off.

In the case of hypoxia, hypoxic injury can be seen as focal loss and holes in kidney capillary balls, from which albumin and red cells will leak, this will result in kidney failure, proteinuria, hematuria + marks, hypoalbuminemia, edema of eyelids, lower limbs and body as well as lumbar and leg soreness and pain. A lot of cold medications, anti-inflammatory painkillers, and medicaments have toxicity for kidney; they can cause damage to the kidney glomerulus, leading to secondary nephrotic syndrome.

Researches in the University of Munster in Germany showed that: in Germany two-thirds of kidney diseases were caused by drug abuse, long-term use of pain reliever, and the body will not produce enough hemoglobin molecule that is needed to carry oxygen, the body hypoxia will furthermore cause kidney disease or kidney failure, and even induce cancer. In short, not only the side effects of the drugs but also the body's drug resistance will eventually affect the body's self-healing ability to repair itself.

To cure kidney disease, renal blood circulation should be improved, firstly to get the urinary filtration function back to normal.

The body self-healing and recuperating shoes can improve renal blood circulation, thereby restoring the normal kidney function of urinary filtration.

The body self-healing and recuperating shoes having medicinal magnetic therapeutic function act on the feet as object of supplementing human bio-electricity, with the shoes as physiotherapy carrier. The shoes are provided with optional number and size of the containers, as a result a special set of energy fields are formed inside the shoes. When the red cells containing ferroprotein circulates with blood flow once in more than 20 seconds passing through the energy field, they cut the magnetic lines of force and thereby resulting in electromagnetic-hydrodynamic phenomena and human bio-electricity. The bio-electricity of 20 trillion human red cells can get 180 times of adequate complement per hour, the degree of excitement and activity of the red cells are enhanced, the speed of their spiral walk will be faster, with their diameter of 8 microns, the red cells can clear capillaries of the diameters of 6-8 micron smoothly one by one, the aged, blocked capillaries in kidneys can be dredged by red cells, the blood circulation in the kidneys will be unobstructed and exuberant, the urinary filtration function will be restored. Lactic acid, uric acid, urea nitrogen, creatinine and other metabolic wastes and toxins will be timely filtered out when the unobstructed and exuberant blood circulation passes through kidney, turn into urine, to be discharged out of the body. For the patients in dialysis, their times of dialysis may be reduced to no more than half than before. When the blood circulation in kidneys is unobstructed and exuberant, there will be no ischemia and hypoxia, kidney disease can be self-healed.

Under the effect of body self-healing and recuperating shoes having medicinal magnetic therapeutical function, human bio-electricity in the red cells will be sufficient to produce enough hemoglobin molecules needed to carry oxygen. More than 1 billion hemoglobin molecules in each red cell can combine with more than 4 billion oxygen molecules, to form oxygenated hemoglobin in lung alveoli, each red cell can carry more than 4 billion oxygen molecules, more than 20 trillion red cells can carry and transport more than 1×10²² oxygen molecules per hour, more than 500 trillion cells in human body can share sufficient supply of more than 1×10²² supplementary oxygen molecules per hour.

With sufficient supply of oxygen, even if injuries and holes developed in the kidney capillary balls, self-healing ability of the surrounding cells of the same type can still play their role, the damaged holes will be subject to regeneration and replacement of kidney blood capillary endothelial cells and epithelial cells in the filtration membrane by proliferative fission of these cells. In that case the integrity of kidney capillary balls can be restored, albumin, red cells will not leak into urine, systemic edema, soreness and pains in the waist and in leg are eliminated, hypoxic lesions such as kidney disease and secondary nephrotic syndrome will get regenerative repaired, and self-healed.

After three months, laboratory tests showed that indices of lactic acid, uric acid, urea nitrogen, creatinine in the urine turn to normal.

### Clinical medicine: the principle that asthma can be recuperated by repairing and enhancing the body self-healing capability:

Systemic hypoxia is the primary cause of asthma attack.

Systemic hypoxia can lead to spasmic contraction of renal artery, reduction of renal blood flow, lowering function of the kidney urinary filtration, causing water and sodium retention, increasing aldosterone, increasing blood volume, thereby increasing pulmonary arterial pressure. Furthermore, it will lead to disruption of lung blood perfusion and obstacle in microcirculation; reduce oxygen uptake capability of lung.

In a state of hypoxia, hypoxic infiltrating and sticking together of eosinophilic granulocytes and lymphocytes among white cells can be observed around the connective tissue mast cells in their respiratory tract in asthma patients' respiratory system, whichever are the types and whatever are the phase of the asthma. Thus immunity of their respiratory system will be lowered, leading to respiratory bacterial infection, hypoxic edema in respiratory mucosa glands, hypertrophy, increased mucus, plasma exudation, recurrent cough, chest distress, and shortness of breath and other inflammatory asthma occurrence and frequent colds. All of the mentioned above will lead to the vicious cycle of the insufficient oxygen in the body.

The human body mainly relies on more than 300 million lung alveoli to absorb oxygen; the total alveolar area is about 100 square meters.

Every gust of cold in the elderly can lead to respiratory infections, this will result in inflammatory damage of a large number of alveolar and hypoxic atrophy and impatency, collapsing into invalid alveolar, reducing alveolar area to contact with oxygen, the decrease of oxygen uptake will lead to gradual aggravation of elderly diseases.

Elderly disease can be divided into 5 major types: hypoxic glucose metabolism disorders such as diabetes; hypoxic cardiovascular diseases such as coronary heart disease, hypertension, cerebral infarction, stroke and others; hypoxic joint type diseases such as cervical and lumbar spondylosis, periarthritis of shoulders, rheumatic and rheumatoid arthritis and others; hypoxic kidney disease; and asthma. The common cause of these five categories of disease suffered by the elderly is hypoxic lesions. Once the elderly suffer from asthma, lung oxygen uptake will be significantly reduced, thereby aggravating the five major categories of illness in elderly patients. The body self-healing and recuperating shoes having medicinal magnetic therapeutic function can restore lung oxygen uptake capability.

The body self-healing and recuperating shoes having medicinal magnetic therapeutic function act on the feet as object of supplementing human bio-electricity, with the shoes as physiotherapy carrier, which are provided with optional number and size of the containers in the shoes, leading to a special set of energy fields inside the shoes. When the red cells containing ferroprotein circulates with blood flow once in more than 20 seconds passing through the energy field, they cut the magnetic lines of force and thereby resulting in electromagnetic hydrodynamic phenomena and human bio-electricity. The bio-electricity of 20 trillion human red cells can get 180 times of adequate complement per hour, the degree of excitement and activity of the red cells are enhanced, the electrophoretic rate will be increased, the speed of their spiral walk will be faster, with their diameter of 8 microns, the red cells can clear capillaries of the diameters of 6-8 micron smoothly one by one, the aged, blocked capillaries in the body can be dredged by red cells, following the red cells, blood perfusion can be fluent in more than 400 million capillaries of the whole body, the human body's blood circulation will be unobstructed and exuberant, the supply of blood to lung can be guaranteed, the oxygen uptake capability can be restored.

Under the effect of body self-healing and recuperating shoes having medicinal magnetic therapeutic function, human bioelectricity in red cells is sufficient, under the effect of electromagnetic hydrodynamics attraction, red cells will be able to absorb and gather sufficient divalent iron ions into the red cell membranes, the red cells can manufacture enough hemoglobin molecules needed for carry oxygen. More than 1 billion hemoglobin molecules in each red cell can combine with more than 4 billion oxygen molecules, to form oxygenated hemoglobin in lung alveoli, each red cell can carry more than 4 billion oxygen molecules, more than 20 trillion red cells can carry and transport more than 1 × 10²² oxygen molecules per hour, more than 500 trillion cells in human body can get sufficient supply of more than 1×10²² supplementary oxygen molecules per hour, the whole body's oxygen supply can be guaranteed. Oxygen supply in whole human body can be guaranteed, blood oxygen concentration in the body will rapidly be enhanced. The source of many diseases, oxygen deficiency, will be eliminated. Hypoxic edema, hyperplasia and hypertrophy and inflammatory injury in respiratory tract mucous glands can get regenerated and self-healed by the respiratory tract mucosa gland cells, lung capillary endothelial cells and alveolar epithelial cells. Hypoxic atrophy and impatency of the pulmonary alveoli can be controlled and improved; the uptake capability of the pulmonary alveoli can basically turn to normal.

Under the effect of body self-healing and recuperating shoes having medicinal magnetic therapeutic function, following unobstructed and exuberant blood circulation, red cells can deliver and supplement sufficient human bio-electricity and oxygen to more than 500 million white cells. The degree of excitement and activity of eosinophils and lymphocytes, among white cells, can be enhanced, they will return to the circulation in lymphatic trunks, lymphatic catheters and veins, hypoxic infiltrating and sticking together is not expected to occur again in respiratory tract, bacteria, viruses and inflammatory pain-causing substances can be swallowed and resolved by leukocytes. Thus, respiratory immune function will return to normal, recurrent cough, chest distress, shortness of breath and asthma can be self-healed; the patient basically does not catch cold. Thus, the problem of aggravating of the diseases suffered by the elderly due to cold-catching has basically been eliminated.

The grounds of reasoning relating to asthma are from clinical medicine all around the world.

### Clinical medicine: hazards of drugs

Pharmacological effects of hypoglycemic drugs:
On the one hand: the hypoglycemic drugs promote the metabolism of glucose in muscle tissue, accelerate blood glucose anaerobic glycolysis. But these effects generate acidic poisons, such as lactic acid, they will bring about poison stimulation to the capillary balls in kidneys. Focal loss of the capillary ball endothelial cells in kidney will happen and damage occurs as holes. Blood glucose will leak from holes on kidney capillary balls into urine, resulting in U-GLU +, kidney failure, and nephrotic syndrome and other severe diseases.

On the other hand, the hypoglycemic drugs inhibit intestinal cells from absorb sugar, to reduce postprandial hyperglycemia. But they also inhibit the intestinal absorption of amino acids. Because all types of human body cells are constructed from proteins with amino acids as the compositions. Over time, the human body will be short of amino acids, leading to hypoproteinemia, emaciation, muscle atrophy, and lower limb edema, as well as fat gluconeogenesis, increased blood glucose, fat metabolism disorder and its accelerated decomposition. Due to the acceleration of the decomposition of fat, large quantities of ketones are produced, also leading to body ketoacidosis and ketone body + marks.

For the diabetic patients, even injection of insulin will only prospone the complications from taking place, diabetic complications such as cardiovascular and cerebrovascular diseases are going to happen sooner or later.

For patients with mild coronary artery disease, normally nothing more than taking drugs can be done to slow down the disease from worsening. But it is only a matter of time for the disease to develop into a severe case with 1-6 stent placed, or 1-3 times heart bypass surgery.

Apart from causing rapid heartbeat, also the drugs of coronary heart disease often lead to increased blood pressure.

The higher the blood pressure is, the more the chances of multiple organ failure are, the shorter the life expectancy is.

When the hypertension is developed to phase III, that is, when the SBP increase to 180 mm Hg, or DBP to 110 mm Hg, myocardial infarction, cerebral infarction, stroke, heart renal failure, uremia are liable to attack with fatality rate of about 30 %. About 70% of patients will possibly suffer from lifelong disabilities such as hemiplegia, paralysis, and failure to take care of themselves all their life.

Diuretics lower blood pressure by diuresis and increasing sodium excretion. Their long-term use can lead to increased secondary aldosterone, hypokalemia, hyperuricemia and hyperglycemia.

Drugs for blood vessels dilation: dilating blood vessels by inhibiting elasticity of the membrane smooth muscle cell in arterial, venous vascular, thereby dilating the blood vessels and lowering the blood pressure. Long-term use can lead to myocaordial ischemia, bradycardia, heart failure, and low blood glucose, reduced elasticity of arteries, elevating the blood vessel brittleness, arteriosclerosis, myocardial infarction, cerebral infarction and stroke.

Experts in the preventive medicine profession believe that while various drugs are treating the disease, their side effects are damaging patients' body function and accelerate the aging of patients, as the cost of seeking temporary equilibrium in the lesion site. Even highly advanced modern medicine does not cure the disease in the true sense; as a result, side effects of drugs accelerate aging of human cells and tissues.

Obviously, all the medications are only helplessly cure the symptoms, not the disease. This is because hypoxia is a common cause of disease in the elderly.

For each elderly patient admitted to hospital, the first measure doctors take is oxygen uptake to enable the patient to ease shortage of oxygen. After the measure is taken, although the disease alleviates, but it is not cured, every time after the patient leaves the hospital; the disease will recur and get worse. This is because that, without eliminating the root cause hypoxia, the elderly disease cannot be cured.

Therefore the oxygen uptake measure hospitals adopt to critically ill patients is an emergency treatment that only cure the symptoms, not the disease, because, when the body bioelectricity is insufficient in red cells, they will not be able to absorb and gather enough divalent iron ions into the cell membranes, thus they cannot produce enough hemoglobin molecules desired to carry oxygen, the number of oxygen molecules carried by red cells is then insufficient. So when the red cells are in shortage of human bio-electricity, even though the patients inhale oxygen every day, oxygen cannot be directly dissolved into blood from the lungs, the body hypoxia problems will not be solved fundamentally.

Experts in preventive medicine profession believe that if we say the side effects of drugs is only to interfere with the normal regulations and balances in the body, then the long-term use of drugs which would generate drug resistance will result in even more diseases that the patient is suffering presently, and even less effective the drugs are. This is because all of the drugs and foods are homologous. Within 15 days the body will generate adaptive and accommodative capability to all drugs, namely: tolerance of drugs and drug resistance generated by the human body. Therefore, after 15 days, most drugs will play a minimal role in the body.

In 2011 baike.baidu.com published a medical paper stating that when the body self-healing capability decline, disease and aging will occur, thus enhancing the body's self-healing capability is the key to the recovery from the diseases. In the process of the rehabilitation of patients, doctors and medicines play less practical role, rehabilitation relies more on self-regulation, self-healing, it is the process of restoring the self-healing capability. Appropriately oxygenating will result in restoring and upgrading body self-healing capability, thereby leading to recovery of the patient.

Under the effect of body self-healing and recuperating shoes having medicinal magnetic therapeutic function, the human bio-electricity in the red cells will be sufficiently supplemented, the red cells will be able to absorb and gather sufficient divalent iron ions into their cell membranes by means of electromagnetic hydrodynamics attraction, they will be able to produce enough hemoglobin molecules needed to carry oxygen. More than 1 billion hemoglobin molecules in each red cell can combine with more than 4 billion oxygen molecules, to form oxygenated hemoglobin in lung alveoli, each red cell can carry more than 4 billion oxygen molecules, more than 20 trillion red cells can carry and transport more than 1×10²² oxygen molecules per hour, more than 500 trillion cells in human body can share sufficient supply of that amount of supplementary oxygen molecules per hour, the whole body's oxygen supply can be guaranteed. Human body self-healing capability can be fully restored and upgraded, hypoxic lesions in human organs and tissues and functional disorders can get regeneratively repaired, various types of hypoxia diseases of elderly can be self-healed. The grounds of reasoning relating to the hazards of drugs are from clinical medicine all around the world.

### Clinical medicine: human tissue cells:

The human body is composed of more than 500 trillion various types of cells, the cell is constituted by proteins, the average diameters of cells are between 10 to 20 microns.

The vast majority of cells in the human body have cycles of declining and regeneration, when some cells in human organs and tissues age and decline, the new cells can be regenerated and replace the old ones. This will always keep the original structures and function of organs and tissues. The life span of human intestinal mucosal cells is three days; that of liver cells is 500 days; those of brain cells and nerve cells in the bone marrow are the same as human lifespan, they are about several decades.

In every minute, more than 100 million cells in human body will wither away and become cell debris, while also more than 100 million new cells will be generated.

Phagocytic white blood cells in the human body have the immune functions such as swallowing and resolving all kinds of inflammatory pain-causing substances, bacteria, viruses, atherosclerotic plaques, cell debris, including thrombus formed by adhesion of cell debris with protein fibers in the blood, etc.

Each leukocyte can swallow and resolve between 5 to 25 bacteria and cell debris. The swallowed and resolved cell debris and bacteria and white blood cells themselves will undergo enzymolysis by lysozyme in the white cell cytoplasm. They will be enzymatically hydrolysed into substances soluble in blood, passing through the kidneys following the blood circulation, and being filtered out, becoming urine excreted out of the body.

The grounds of reasoning relating to human tissues and cells are from clinical medicine all around the world.

### Clinical medicine: cell regeneration:

Physiological regeneration: under normal physiological conditions, when some of the tissue cells cyclically decline with the aging of cells, the regeneration constantly supplements the human body with new-born cells to tissues of the same types, maintaining the integrity of the original human organs and tissues.

Pathological regeneration: when body lesions occur in pathological states, defect or necrosis emerge in some of the cells in human organs and tissues, then same kind of surrounding cells can proliferate by splitting, differentiation and regeneration, supplementing these new-born cells in a timely and recycling manner, it can completely restore the original structures and function of human organs and tissues. The organs still cannot regenerate can still be repaired by granulation tissues and scars.

However, these pathological regenerations are completed only when the human body in a state of adequate oxygen content in the body.

In the stated of hypoxia, when there are defects or necrotic cells in human organs and tissues, owing to lack of proliferation, division, differentiation regeneration, the original structure and function of human organs and tissues cannot be restored.

### Method of application of the energy cartridges and catalytic solution:

Method of application of energy cartridge and the catalyst solution [For 1 course/30 days per pack]:
① The energy cartridges placed in each pair of shoes are divided into: large, medium and small sized; 6 boxes totally. The energy cartridge should be replaced once in six days.
② Before use, first take out energy cartridges of large, medium and small sizes, 2 boxes each, and place the 2 pieces of spare magnetic sheets in the recessed groove below large energy cartridges, pasted them up with scotch tape, so as not to lose them. Then the large, medium and small cartridges each placed in a fixed position in the corresponding recessed grooves inside the shoe with fabric upwards.
③ Every time when energy cartridges are replaced, remove the magnetic sheets from under the already used large energy cartridges; re-paste them to the recessed grooves under the new large energy cartridges to continue using them. In order to facilitate removal, small strips of cloth can be padded under the cartridges; pulling one end of the cloth can easily remove the cartridges.
④ Every morning the catalytic solution should be dropped into large, medium and small energy cartridges, respectively, to soak the dry powder in the energy cartridges, to an appropriate extent that the liquid will not discharge at the foot pressure. When the discharge occurs the catalyst solution should be wiped out. If you drop too much catalyst solution, it will bring about stimulation to the feet, causing blisters or ulceration.
⑤ Every time before use of the catalyst liquid, please shake it vigorously for 10 times to homogenize it.

The energy cartridges and catalyst solution in any number and size of container set up by the body self-healing and recuperating shoes are special preparations among ones of the kind all over the world that do not add any preservatives or fungicides, also it does not generate a single pathogen.

The energy cartridges and catalyst solution have effects of promoting blood circulation, removing blood stasis, promoting recovery, anti-inflammatory and analgesic, detoxification and relieving itch, can promote human body regeneration and enhancement of the self-healing function. It can promote unobstructed and exuberant blood circulation, enhance the viability of variable human cell types, and enhance human body immune function.

Applying catalyst solution to the skin for about five days, problems such as traumatic bleeding, swelling and inflammation, all kinds of infections, skin fissures in hands and feet, skin rashes, herpes, frostbite and acne in the skin can be rapidly eliminated and self-healed.

### Requirements on stopping using various types of medicines, the energy cartridges and catalytic solution:

① All patients who are taking hypoglycemic medicines, injecting insulin, taking hypotensive medicines, cardio-cerebrovascular medicines, and hormone medicines should be noted: at the beginning of wearing the shoes, administration of these drugs should not be suspended in order to avoid accidents. After using the energy cartridges and catalytic solution for 3 months, then according to the improvement of the symptoms, gradually the amount of the above mentioned medicines taken may be decreased.
② When the blood glucose, blood pressure, heartbeat recovers to normal, and all kinds of symptoms are eliminated, the use of energy cartridges and catalytic liquid should be continued to be used for a furthermore course for consolidation, then the shoes can be put on as ordinary shoes.

### Precautions:

① The body self-healing and recuperating shoes should be put on for more than a total of 10 hours daily. Their daily use should not be interrupted.
② Diabetic patients should choose loose shoes that fit the feet perfectly, in order to avoid rubbing the feet and subsequently infection fester or other consequences. Diabetic patients are not sensitive to temperature, so it is forbidden to use more than 40°C hot water to bath the feet in order to avoid the feet scalded and other problems such as blistering, infection, gangrene, sepsis, or even amputation.
③ Diabetes patients with complications of severe muscle atrophy, cataract, retinal detachment, blindness can still wear the body self-healing and recuperating shoes, which will not bring about any adverse reaction to the above lesions. But these severe lesions are not included in the self-healing ranges by means of the body self-healing and recuperating shoes.
④ Do not pad other insoles or wear thick socks, in order to avoid reducing the effect.
⑤ Soles should be checked daily to avoid metal objects adsorbed to soles, leading to stabbing in the feet.

### Prohibitions:

① pregnant and lactating women, people with spiritual and mental retardation.
② patients with severe bleeding in hemorrhage period.
③ patients who are suffering from following diseases: diabetic ketoacidosis, foot ulcers, combined with congestive heart failure having a history of I-IV grade critical illness, and cancer.

## Claims

1. A pair of body self-healing and recuperating shoes having a medicinal magnetic therapeutic function, wherein one or more breathable containers are set up in said recuperating shoes, said containers contain: a volatile oily medicament, a non-volatile oily liquid medicament or a powdered medicament;
said volatile oily medicament comprises 1-9 parts by weight of the following raw medicines: angelica anomala oil or its extract, or clove oil or its extract, and 1-9 parts by weight of borneol corresponding to each of the raw medicine;
said powdered medicament comprises 1-9 parts by weight of the following raw medicines: ligusticum wallichii, abutilon pepper, amomum cardamom, angelica anomala and clove, and 1-9 parts by weight of borneol corresponding to each of the raw medicine;
said non-volatile oily liquid medicament comprises 1-9 parts by weight of the following raw medicines: ligusticum wallichii, abutilon pepper, amomum cardamom, angelica anomala and clove, and 1-9 parts by weight of borneol corresponding to each of the raw medicine;
one or more magnets are installed in said body self-healing and recuperating shoes; wherein said magnets are installed in the sole and/or upper and/or back counter of the shoes, and said breathable containers are located within the insole;
wherein said magnets are magnetic beads, one or more recessed grooves are cut into said shoe soles or insoles, a magnetic bead is set up in each recessed groove; the length, width and height of the recessed groove is greater than the diameter of the magnetic bead, allowing the bead to roll freely.

2. The body self-healing and recuperating shoes having a medicinal magnetic therapeutic function according to claim 1, wherein said container is punched with holes or covered with cloth, allowing the medicaments to evaporate out from the holes or cloth.

3. The body self-healing and recuperating shoes having a medicinal magnetic therapeutic function according to claim 1, wherein said body self-healing and recuperating shoes include the shoes suitable for wearing during spring or fall seasons, sandals, slippers and cotton-padded shoes.

4. The body self-healing and recuperating shoes having a medicinal magnetic therapeutic function according to claim 1, wherein said single volatile oily medicament is prepared by adding borneol into said raw medicines; then the mixture of raw medicines can be obtained by mixing single volatile oily medicaments with each other.

5. A method of preparation of the powdered medicament of claim 1, wherein:
(1) ligusticum wallichii, abutilon pepper, amomum cardamom, angelica anomala and clove, and 1-9 parts by weight of borneol corresponding to each of the raw medicine are weigh out according to the weight ratio;
(2) Pick and grind each of the above-mentioned raw materials into powders and sieve the powders to obtain the respective raw medicinal powders;
(3) Add borneol into each raw medicinal powder to obtain said single raw medicinal powder, then the mixture of the raw medicinal powders can be obtained by mixing respective single raw medicinal powders with each other.

6. A method of preparation of the non-volatile oily liquid medicament of claim 1, wherein:
(1) weigh out the raw materials according to the weight ratio of 1-9 parts by weight of one of the following raw medicines: angelica anomala oil or its extract, or clove oil or its extract, and 1-9 parts by weight of borneol corresponding to each of the raw medicine;
(2) Pick carefully and grind each of the above-mentioned raw materials into the powders and sieve the powders to obtain the respective raw medicinal powders;
(3) Add borneol into each raw medicinal powder to obtain said single raw medicinal powders;
(4) Soak each of said single raw medicinal powder in an acidic solution respectively, then extract the powders by diacolation to obtain extracted stock solutions of single raw medicinal powders, then mixed the single extracted stock solutions with each other.

7. The method according to claim : 6, wherein said acidic solution is acetic acid.

8. The body self-healing and recuperating shoes having a medicinal magnetic therapeutic function according to claim 1, wherein the recessed groove can be ring-, oval ring-, rectangular-ambulatory-plane-, cross-, long strip- or triangle shaped; said oval ring-, rectangular-ambulatory-plane-, cross shaped-, long strip- or triangle-shaped recessed grooves can be arranged in longitudinal, transverse or diagonal directions.

9. The body self-healing and recuperating shoes having a medicinal magnetic therapeutic function according to claim 1, wherein more than one shallow recessed groove, or more than one pair of parallel shallow recessed grooves are set up in said portions of the soles or insoles in contact with the soles of the feet, as well as in the upper- and back counter of the shoes; more than one magnet or more than one pair of parallel magnets are set up in each recessed groove.

10. The body self-healing and recuperating shoes having a medicinal magnetic therapeutic function according to claim 1, wherein the magnets are selected from rare earth permanent magnetic materials, or various types of hard magnets containing magnetic materials, and soft magnets made of rubber or plastic or other soft materials.

## Patentansprüche

1. Schuhe mit selbstheilender und regenerierender Wirkung auf den Körper mit einer medizinischen magnetischen therapeutischen Funktion, wobei ein oder mehrere atmungsaktive Behälter in den Schuhen mit regenerierender Wirkung vorgesehen sind, wobei die Behälter enthalten: ein flüchtiges ölhaltiges Arzneimittel, ein nichtflüchtiges ölhaltiges flüssiges Arzneimittel oder ein pulverförmiges Arzneimittel,
wobei das flüchtige ölhaltige Arzneimittel 1 bis 9 Gewichtsteile der folgenden Roharzneimittel: Öl von Angelica anomala oder sein Extrakt oder Nelkenöl oder sein Extrakt und 1 bis 9 Gewichtsteile Borneol entsprechend jedem der Roharzneimittel enthält,
wobei das pulverförmige Arzneimittel 1 bis 9 Gewichtsteile der folgenden Roharzneimittel: Ligusticum wallichii, Abutilon-Pfeffer, Amomum Cardamom, Angelica anomala und Nelke sowie 1 bis 9 Gewichtsteile Borneol entsprechend jedem der Roharzneimittel enthält,
wobei das nichtflüchtige ölhaltige flüssige Arzneimittel 1 bis 9 Gewichtsteile der folgenden Roharzneimittel: Ligusticum wallichii, Abutilon-Pfeffer, Amomum Cardamom, Angelica Anomala und Nelke und 1 bis 9 Gewichtsteile Borneol entsprechend jedem der Roharzneimittel enthält,
wobei ein oder mehrere Magnete in den Schuhen mit selbstheilender und regenerierender Wirkung auf den Körper installiert sind, wobei die Magnete in der Sohle und/oder im oberen und/oder im hinteren Teil der Schuhe installiert sind und die atmungsaktiven Behälter innerhalb der Einlegesohle angeordnet sind,
wobei die Magnete Magnetkugeln sind, eine oder mehrere vertiefte Nuten in den Schuhsohlen oder Einlegesohlen ausgeschnitten sind und in jeder vertieften Nut eine Magnetkugel eingesetzt ist, wobei die Länge, die Breite und die Höhe der vertieften Nut größer sind als der Durchmesser der Magnetkugel, so dass die Kugel frei rollen kann.

2. Schuhe nach Anspruch 1, wobei der Behälter mit Löchern gestanzt oder mit einem Tuch bedeckt ist, wodurch ermöglicht wird, dass die Arzneimittel von den Löchern oder dem Tuch daraus verdunsten können.

3. Schuhe nach Anspruch 1, wobei die Schuhe mit selbstheilender und erholender Wirkung für den Körper Schuhe, die zum Tragen während der Frühlings- oder Herbstsaison geeignet sind, Sandalen, Hausschuhe oder mit Baumwolle gefütterte Schuhe sind.

4. Schuhe nach Anspruch 1, wobei das einzelne flüchtige ölhaltige Arzneimittel durch Zugabe von Borneol zu den Roharzneimitteln hergestellt wird und anschließend eine Mischung von Roharzneimitteln durch Mischen einzelner flüchtiger ölhaltiger Arzneimittel miteinander erhalten werden kann.

5. Verfahren zum Herstellung des pulverförmigen Arzneimittels nach Anspruch 1, wobei:
(1) Ligusticum wallichii, Abutilon-Pfeffer, Amomum Cardamom, Angelica anomala und Nelke sowie 1 bis 9 Gewichtsteile Borneol entsprechend jedem der Roharzneimittel gemäß dem Gewichtsverhältnis abgewogen werden;
(2) jedes der vorstehend erwähnten Rohmaterialien ausgewählt und zu Pulver gemahlen wird und das Pulver gesiebt wird, um die jeweiligen medizinischen Rohpulver zu erhalten;
(3) Borneol zu jedem medizinischen Rohpulver hinzugefügt wird, um das einzelne medizinische Rohpulver zu erhalten, woraufhin die Mischung der medizinischen Rohpulver erhalten werden kann, indem entsprechende einzelne medizinische Rohpulver miteinander gemischt werden.

6. Verfahren nach Anspruch 1, wobei:
(1) die Rohmaterialien gemäß dem Gewichtsverhältnis von 1 bis 9 Gewichtsteilen eines der folgenden Roharzneimittel abgewogen werden: Öl von Angelica anomala oder sein Extrakt oder Nelkenöl oder sein Extrakt und 1 bis 9 Gewichtsprozent Borneol entsprechend jedem der Roharzneimittel;
(2) jedes der vorstehend erwähnten Rohmaterialien sorgfältig ausgewählt und zu einem Pulver gemahlen wird und die Pulver gesiebt werden, um die jeweiligen medizinischen Rohpulver zu erhalten;
(3) Borneol jedem medizinischen Rohpulver hinzugefügt wird, um die einzelnen medizinischen Rohpulver zu erhalten;
(4) jedes der einzelnen medizinischen Rohpulver in einer sauren Lösung eingetaucht wird, dann die Pulver durch Diakolation extrahiert werden, um extrahierte Stammlösungen einzelner medizinischer Rohpulver zu erhalten, und dann die einzelnen extrahierten Stammlösungen miteinander gemischt werden.

7. Verfahren nach Anspruch 6, wobei die saure Lösung Essigsäure ist.

8. Schuhe nach Anspruch 1, wobei die vertiefte Nut ringförmig, oval-ringförmig, rectangular-ambulatory-plane, kreuzförmig, langstreifenförmig oder dreieckig ausgebildet sein kann, und wobei die oval-ringförmigen, rectangular-ambulatory-plane, kreuzförmigen, langstreifenförmigen oder dreieckigen vertieften Nuten in Längs-, Quer- oder Diagonalrichtung angeordnet sein können.

9. Schuhe nach Anspruch 1, wobei mehr als eine flache vertiefte Nut oder mehr als ein Paar parallele flache vertiefte Nuten in den Abschnitten der Sohlen oder Einlegesohlen angeordnet sind, die mit den Fußsohlen in Kontakt stehen, sowie im oberen und hinteren Teil der Schuhe, und wobei in jeder vertieften Nut mehr als ein Magnet oder mehr als ein Paar parallele Magnete angeordnet sind.

10. Schuhe nach Anspruch 1, wobei die Magnete ausgewählt sind aus Seltenerd-Permanentmagnetmaterialien oder verschiedenen Arten von harten Magneten, die magnetische Materialien enthalten, und weichen Magneten, die aus Gummi oder Kunststoff oder anderen weichen Materialien hergestellt sind.

## Revendications

1. Paire de chaussures corporelles auto-cicatrisantes et de récupération ayant une fonction thérapeutique magnétique médicale, où un ou plusieurs récipients respirants sont installés dans lesdites chaussures de récupération, lesdits récipients contiennent : un médicament huileux volatil, un médicament liquide huileux non volatil ou un médicament en poudre ;
ledit médicament huileux volatil comprend 1 à 9 parties en poids des produits médicinaux bruts suivants : de l'huile d'angelica anomala ou son extrait, de l'huile de clou de girofle ou son extrait, et 1 à 9 parties en poids de bornéol correspondant à chaque produit médicinal brut ;
ledit médicament en poudre comprend 1 à 9 parties en poids des produits médicinaux bruts suivants : le ligusticum wallichi, l'abutilon pepper, l'amomum cardamom, l'angelica anomala et clou de girofle, et 1 à 9 parties en poids de bornéol correspondant à chaque produit médicinal brut ;
ledit médicament liquide huileux non volatil comprend 1 à 9 parties en poids des produits médicinaux bruts suivants : le ligusticum wallichi, l'abutilon pepper, l'amomum cardamom, l'angelica anomala et le clou de girofle, et 1 à 9 parties en poids de bornéol correspondant à chaque produit médicinal brut ;
un ou plusieurs aimants sont installés dans lesdites chaussures corporelles auto-cicatrisantes et de récupération : où lesdits aimants sont installés dans la semelle et/ou dans le contrefort supérieur et/ou arrière des chaussures et lesdits récipients respirants sont situés à l'intérieur de la semelle intérieure ;
où lesdits aimants sont des billes magnétiques, une ou plusieurs rainures en retrait sont découpées dans lesdites semelles ou semelles intérieures de la chaussure, une bille magnétique est installée dans chaque rainure en retrait ; la longueur, la largeur et la hauteur de la rainure en retrait sont supérieurs au diamètre de la bille magnétique, permettant à la bille de rouler librement.

2. Paire de chaussures corporelles auto-cicatrisantes et de récupération ayant une fonction thérapeutique magnétique médicale selon la revendication 1, dans laquelle ledit récipient est perforé avec des trous ou recouvert d'un tissu, permettant aux médicaments de s'évaporer hors de trous ou du tissu.

3. Paire de chaussures corporelles auto-cicatrisantes et de récupération ayant une fonction thérapeutique magnétique médicale selon la revendication 1, où lesdites chaussures corporelles auto-cicatrisantes et de récupération comportent les chaussures appropriées pour être portées pendant les saisons du printemps ou de l'automne, les sandales, les pantoufles et des chaussures rembourrées de coton.

4. Paire de chaussures corporelles auto-cicatrisantes et de récupération ayant une fonction thérapeutique magnétique médicale selon la revendication 1, dans laquelle ledit médicament huileux volatil seul est préparé en ajoutant du bornéol dans lesdits produits médicinaux bruts ; puis le mélange de produits médicinaux bruts peut être obtenu en mélangeant des médicaments huileux volatils seuls les uns avec les autres.

5. Procédé de préparation d'un médicament en poudre selon la revendication 1, dans lequel :
(1) du ligusticum wallichii, de l'abutilon pepper, de l'amomum cardamom, de l'angelica anomala et du clou de girofle, et 1 à 9 parties en poids de bornéol correspondant à chacun des produits médicinaux bruts sont pesés selon le ratio en poids ;
(2) le prélèvement et la mouture de chacune des matières brutes mentionnées ci-dessus sous forme de poudres et le tamisage des poudres pour obtenir les poudres médicinales brutes respectives ;
(3) l'ajout de bornéol à chaque poudre médicinale brute pour obtenir ladite poudre médicinale brute seule, ensuite le mélange des poudres médicinales brutes peut être obtenu en mélangeant les poudres médicinales brutes seules respectives les unes avec les autres.

6. Procédé de préparation d'un médicament liquide huileux non volatil selon la revendication 1, dans lequel :
(1) on pèse les matières brutes selon le ration en poids de 1 à 9 parties en poids de l'un des produits médicinaux bruts suivants : l'huile d'angelica anomala ou son extrait, ou l'huile de clou de girofle ou son extrait, et 1 à 9 parties en poids de bornéol correspondant à chaque produit médicinal brut ;
(2) on prélève soigneusement et on effectue la mouture de chacune des matières brutes mentionnées ci-dessus en poudres et on tamise les poudres pour obtenir les poudres médicinales brutes respectives ;
(3) on ajoute du bornéol à chaque poudre médicinale brute pour obtenir lesdites poudres médicinales brutes seules ;
(4) on saponifie chacune des dites poudres médicinale brute seule dans une solution acide, respectivement, puis on extrait les poudres par une diacolation pour obtenir des solutions mères de poudres médicinales brutes seules, puis on mélange les solutions mères extraites seules les unes avec les autres.

7. Procédé selon la revendication 6, dans lequel la solution acide est de l'acide acétique.

8. Paire de chaussures corporelles auto-cicatrisantes et de récupération ayant une fonction thérapeutique magnétique médicale selon la revendication 1, dans laquelle la rainure en retrait peut être de forme annulaire, annulaire ovale, plane ambulatoire rectangulaire, en forme de croix, en forme de bande longue ou en triangle ; lesdites rainures en retrait de forme annulaire, annulaire ovale, plane ambulatoire rectangulaire, en forme de croix, en forme de bande longue ou en triangle peuvent être agencées dans des directions longitudinales, transversales ou diagonales.

9. Paire de chaussures corporelles auto-cicatrisantes et de récupération ayant une fonction thérapeutique magnétique médicale selon la revendication 1, dans laquelle plus d'une rainure en retrait peu profonde, ou plus d'une paire de rainures en retrait peu profondes parallèles sont installées dans lesdites parties des semelles ou des semelles intérieures en contact avec les semelles des pieds, ainsi que dans le contrefort supérieur et arrière des chaussures ; plus d'un aimant ou plus d'une paire d'aimants parallèles sont installés dans chaque rainure en retrait.

10. Paire de chaussures corporelles auto-cicatrisantes et de récupération ayant une fonction thérapeutique magnétique médicale selon la revendication 1, dans laquelle les aimants sont choisis parmi des matériaux magnétiques permanents de terres rares, ou des types variés d'aimants durs contenant des matériaux magnétiques, et des aimants doux fabriqués à base de caoutchouc ou de matières plastiques ou d'autres matériaux mous.
